# EUROPEAN PATENT SPECIFICATION

(11) **EP 3 481 345 B1**
(45) Date of publication and mention of the grant of the patent: **14.01.2026**
(21) Application number: 17742559.2
(22) Date of filing: 07.07.2017
(51) Int. Cl.: A61F 5/56

(54) **DENTAL APPLIANCE FOR SLEEP APNEA, SNORING AND TONGUE AND ORAL CAVITY REMODELING**
DENTALGERÄT GEGEN SCHLAFAPNOE, SCHNARCHEN UND ZUNGEN- UND MUNDHÖHLENREMODELLIERUNG
APPAREIL DENTAIRE CONTRE L'APNÉE DU SOMMEIL ET LES RONFLEMENTS ET POUR LA RÉORGANISATION DE LA LANGUE ET DE LA CAVITÉ BUCCALE

(30) Priority: 08.07.2016 US 201662360230 P
(43) Date of publication of application: 15.05.2019
(73) Proprietor: Tongue Lab Europe Ltd., Fulham, London SW6 4TU (GB)
(72) Inventor: MAUCLAIRE, Claude, 10000 Troyes (FR); VANPOULLE, Frederic, 75005 Paris (FR); QUESTEL,Sebastien Corlay, 75005 Paris (FR); MAUCLAIRE, Jean-Michel, 75005 Paris (FR)
(74) Representative: Marks & Clerk LLP
(86) International application number: PCT/IB2017/054130
(87) International publication number: WO 2018/008002

(56) References cited:
- US-A1- 2004 049 102
- US-A1- 2010 288 288
- US-A1- 2013 125 902

## Description

### TECHNICAL FIELD

The present disclosure relates to the field of dental appliances, and more specifically to a dental appliance for restraining and retraining the tongue.

### BACKGROUND

Dental appliances, including orthodontic appliances, have been used for many years to correct various malformations and conditions related to the teeth and jaw. However, many of these dental appliances have not adequately addressed the role of the tongue in the occurrence and persistence of certain dental, mandibular and palatal conditions.

The tongue includes a pharyngeal portion and a buccal portion. The pharyngeal portion begins at the hyoid bone and connects to the skull, and the pharynx, while the buccal portion connects to the front portion of the lower jaw and is the outwardly visible portion of the tongue, namely the tip of the tongue, an anterior central zone of the tongue, and the dorsum of the tongue.

For various reasons, including congenital defects and/or habits developed during childhood (e.g., transitioning from sucking to learning how to chew, learning how to speak), the tongue assumes an incorrect posture within the mouth. As a result, the tongue is in an abnormal position-a condition known as tongue dysfunction. Tongue dysfunction can sometimes lead to the development of other oral and mandibular conditions such as malocclusion of the teeth or labioversion of certain teeth. Tongue dysfunction can also contribute to the development of sleep breathing disorders such as snoring and sleep apnea. More specifically, a tongue with an inappropriate resting position fails to enlarge the palate. In turn, this causes the palate to become hollow, which reduces the volume of the nasal fossae and can narrow the pharynx and more generally the upper airway. This can result in reduced respiration through the nose, and thus forced respiration through the mouth (mouth breathing). In other cases, the tongue, resting backward and high against the soft palate, reduces the size of or even obstructs the pharynx. This narrowing of the respiratory airways can lead to excessive snoring and sometimes obstructive sleep apnea, a condition in which a person stops breathing for one or more time periods during sleep. Obstructive sleep apnea can have moderate to severe effects, including fatigue, somnolence, chronic headaches, and cardiovascular and metabolic pathologies in the most severe cases.

Current strategies for reducing tongue dysfunction involve training exercises. More commonly, treatment of its associated medical conditions primarily involves use of various treatment procedures including orthodontics. Both training and orthodontic procedures, however, are time-consuming, costly, have varying levels of success and often result in relapse of the condition. Among the causes of these relapses is the failure to fully retrain the tongue. Without retraining of the tongue, including after surgery, to modify improper movements, the patient will reflexively continue to move their tongue in the same manner and permit it to rest improperly as they did prior to the treatment.

As such, there is a need for apparatuses and methods of training or retraining the tongue to correct or alleviate symptoms associated with tongue dysfunction. There is also a need for apparatuses and methods for training the tongue to correct tongue dysfunction and alleviate buccal structural defects associated with the dysfunction without orthodontics.

The applicant has made progress in alleviating the foregoing abnormalities by devising appliances for constraining the tongue described in:
(i) US Patent Publication 20110284011 (US Patent Application No 12/976489) filed on December 22, 2010 as a c-i-p of PCT/EP2009/060226 filed August 6, 2009, in turn claiming the priority of US provisional application 61/086,684 filed August 6, 2008; and
(ii) US Patent Publication 20110262881 (US Patent Application No 13/057141) filed on June 3, 20111 as a national stage application of the foregoing PCT application and claiming the priority of the same provisional application.

US 2004/0049102 discloses a system for stabilizing a tongue in an oral cavity comprising an operative element sized and configured to be fitted to less mobile tissue in the oral cavity, the operative element being further constructed and arranged to interact with a tongue in the airway to stabilize a preferred tissue orientation.

The present devices represent improvements to the appliances described in the foregoing earlier patent publications in design, appearance and functionality For example, the prior devices were early versions and were removable only at a dental professional's office; they featured attachment to molar (or other rear) teeth by a band encircling the teeth or other cumbersome means, including (in some embodiments) means that interfered with complete occlusion; and/or while adjustable, adjustment, especially repeated adjustment, could cause the device to break and it then had to be replaced. The devices often relied on sheaths for securing the component to the attachment mechanism and had a prototype-like appearance that presented a distraction for professionals wearing the device in business meetings and more generally in a business environment. Adjustments were also very difficult to make during treatment.

### SUMMARY

The present invention relates to a dental appliance as set out in claim 1. Preferred embodiments are set out in the dependent claims.

### BRIEF DESCRIPTION OF THE DRAWINGS

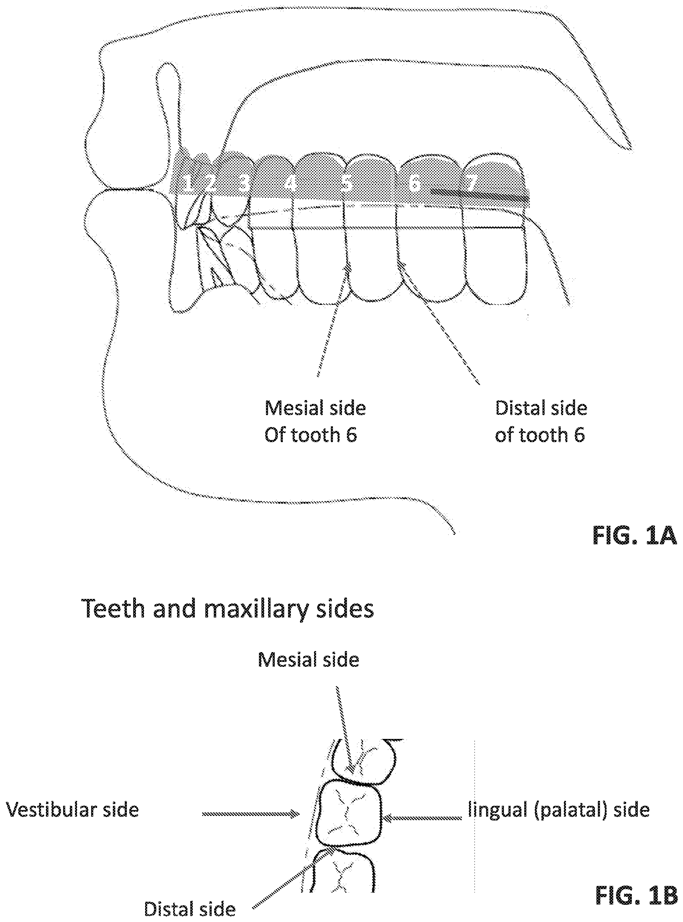

FIGS. 1A-B show various aspects of the dental arcade of a person and illustrate nomenclature used herein;

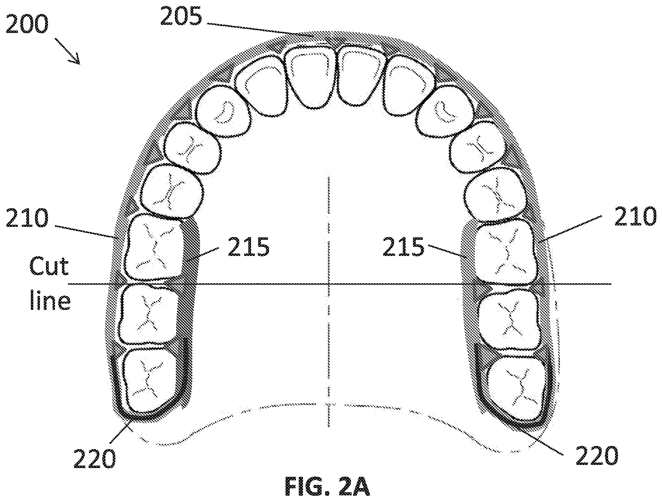

FIGS. 2A-C show a bottom view (2A) a lingual side view (2B), and a vestibular side view (2C) of the attachment mechanism of a dental appliance on the top dental arcade in accordance with one or more embodiments;

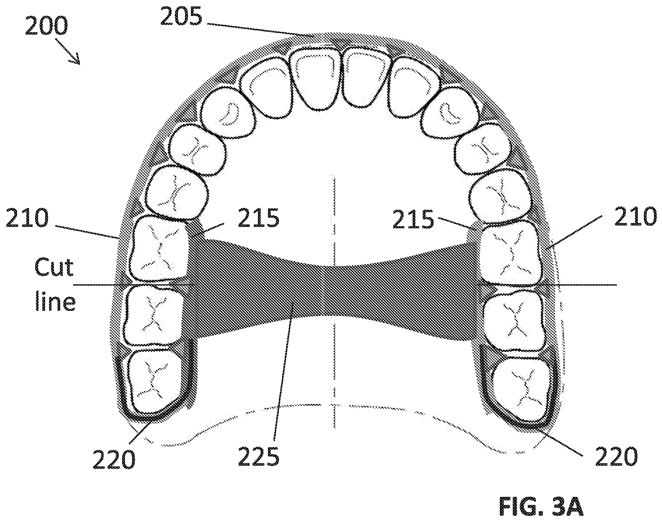

FIGS. 3A-C show a bottom view (3A), a lingual side view (3B), and a vestibular side view (3C) of an embodiment of the attachment mechanism further having an (optional) arcuate support member, in accordance with one or more embodiments;

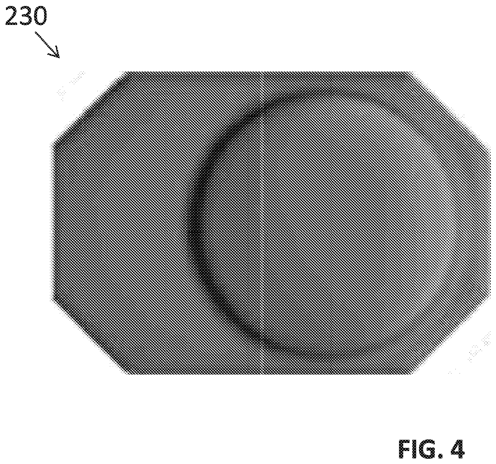

FIG. 4 shows an exemplary sensing device for attachment to the dental appliance in accordance with one or more embodiments;

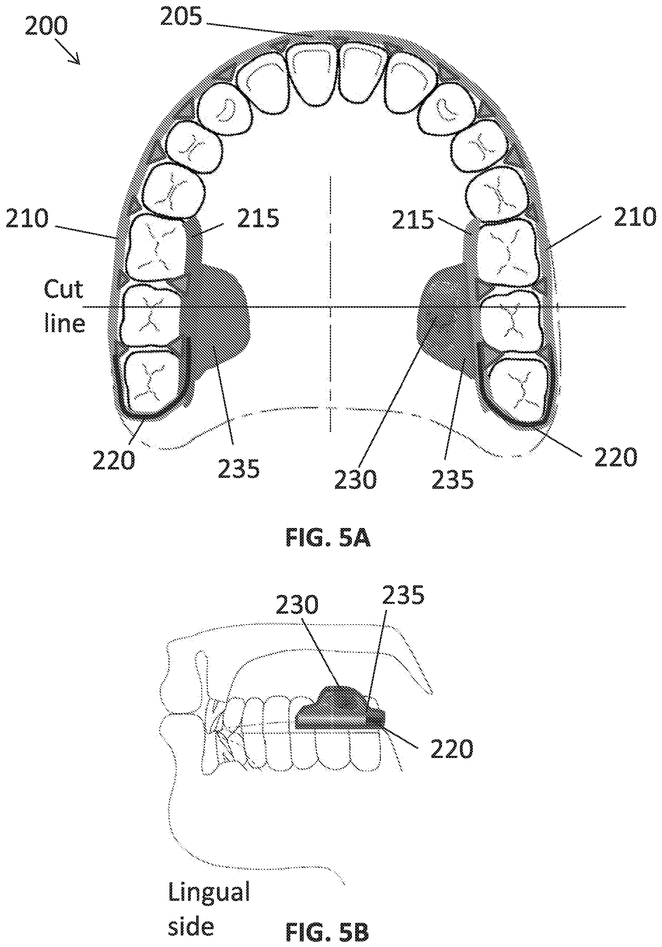

FIGS. 5A-B show a bottom view (5A) and a lingual side view (5B) an embodiment of the attachment mechanism having one or, for balance, two wings and a sensing device embedded in one wing (or optionally different devices embedded in one or both wings) in accordance with one or more embodiments;

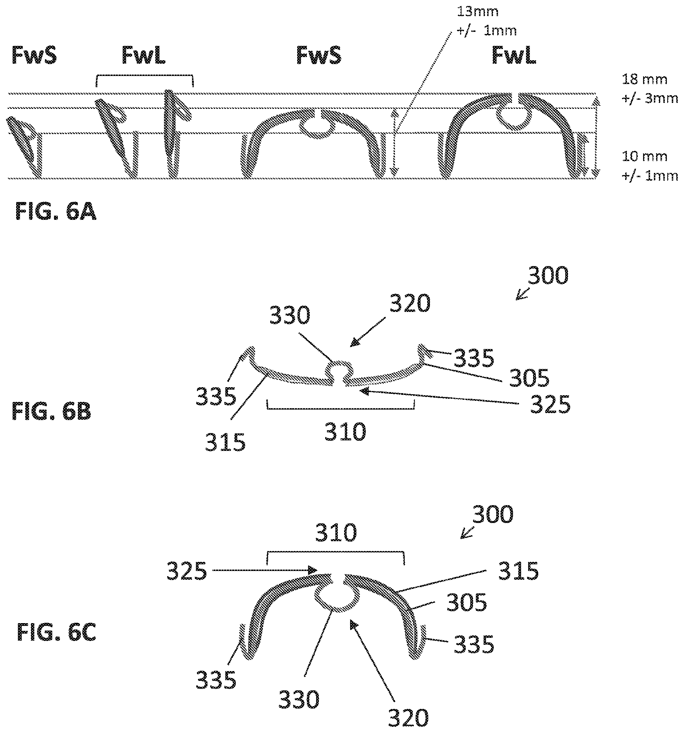

FIGS. 6A-C show exemplary constraining mechanisms having a forward orientation and a central buckle in accordance with one or more embodiments. FIG. 6A shows exemplary lengths of a forward-oriented short constraining mechanism (FwS) and a forward-oriented long constraining mechanism (FwL) in accordance with one or more embodiments. FIG. 6B shows a front view of an exemplary forward-oriented constraining mechanism, and FIG. 6C shows a rear view of an exemplary forward-oriented constraining mechanism in accordance with one or more embodiments;

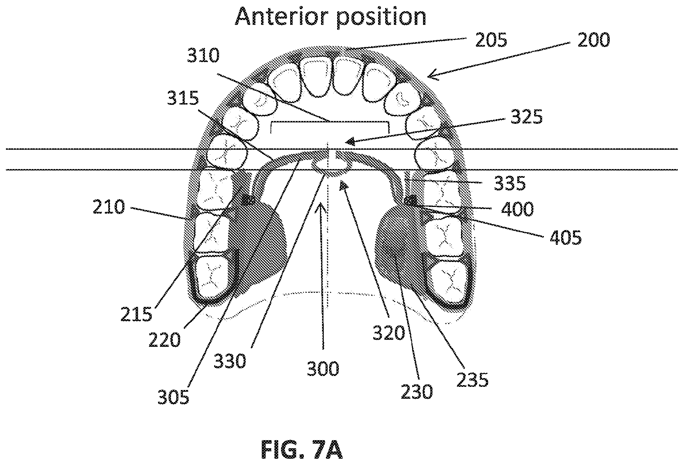

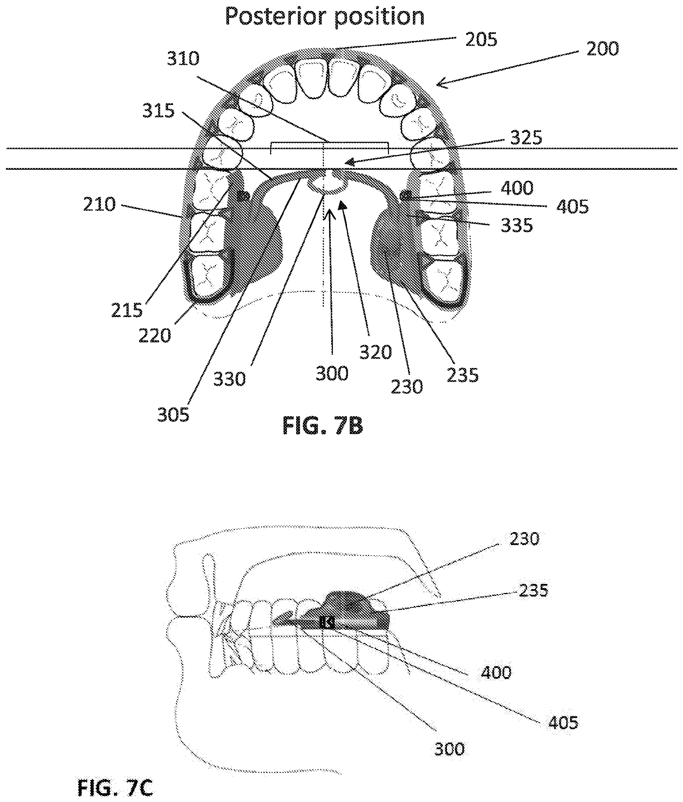

FIGS. 7A-C show an exemplary constraining mechanism having a forward orientation and its attachment to an attachment mechanism having wings via a fastening mechanism in accordance with one or more embodiments. FIG. 7A shows a bottom view in which the constraining mechanism is in an anterior position, FIG. 7B shows a bottom view in which the constraining mechanism is in a posterior position, and FIG. 7C shows a lingual side view in which the constraining mechanism is in an anterior position;

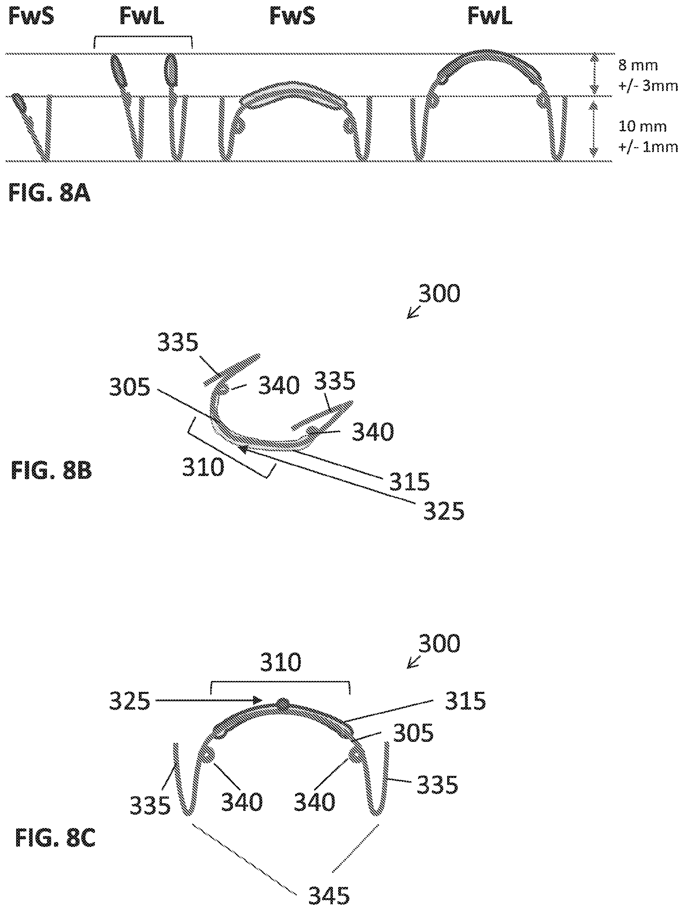

FIGS. 8A-C show exemplary constraining mechanisms having a forward orientation and horizontal loops in accordance with one or more embodiments. FIG. 8A shows exemplary lengths of a forward-oriented short constraining mechanism (FwS) and a forward-oriented long constraining mechanism (FwL) in accordance with one or more embodiments. FIG. 8B shows a front perspective view of the exemplary forward-oriented constraining mechanism with horizontal loops, and FIG. 8C shows a rear view of the exemplary forward-oriented constraining mechanism in accordance with one or more embodiments;

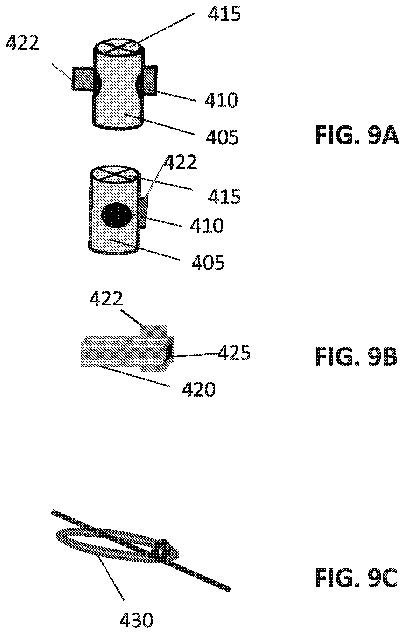

FIGS. 9A-C show aspects of exemplary fastening (including tightening) mechanisms and adjustment mechanisms (for back and forth adjustment of the position of the constraining mechanism) in accordance with one or more embodiments;

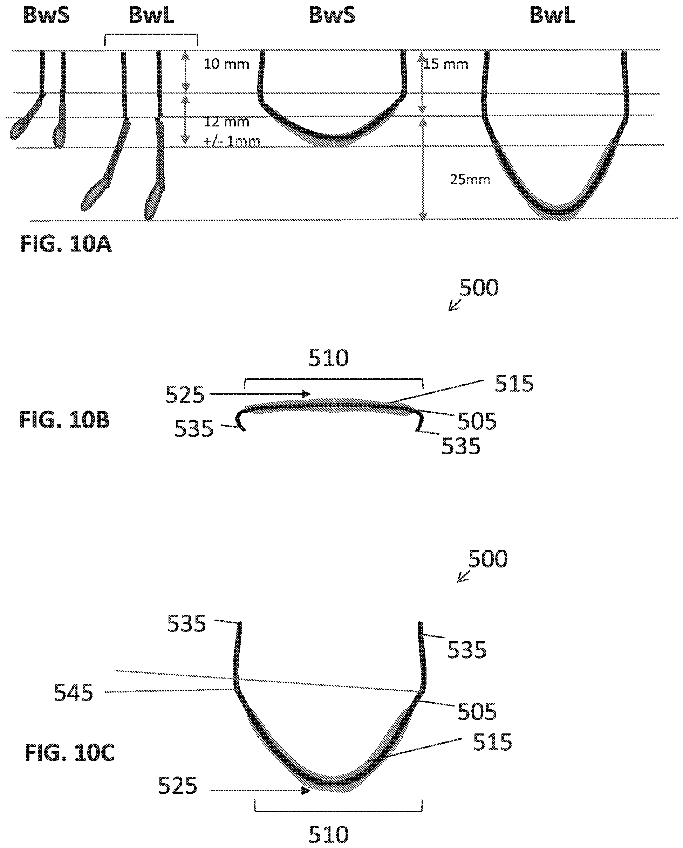

FIGS. 10A-C show exemplary constraining mechanisms having a backward (rearward) orientation in accordance with one or more embodiments. FIG. 10A shows exemplary lengths of a backward-oriented short constraining mechanism (BwS) and a backward-oriented long constraining mechanism (BwL) in accordance with one or more embodiments. FIG. 10B shows a front view of the exemplary backward-oriented constraining mechanism, and FIG. 10C shows a top view of the exemplary backward-oriented constraining mechanism in accordance with one or more embodiments;

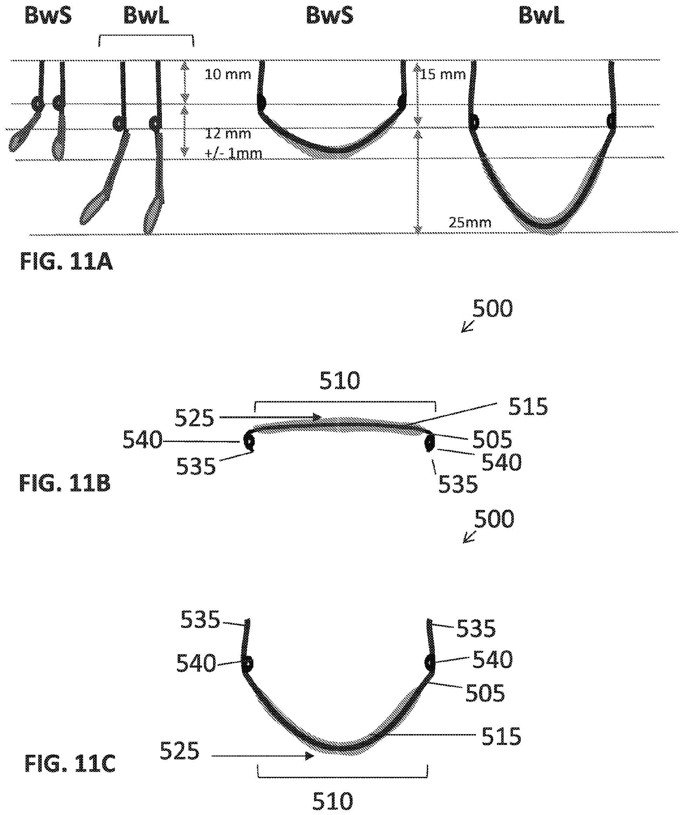

FIGS. 11A-C show exemplary constraining mechanisms having a backward orientation and horizontal loops in accordance with one or more embodiments. FIG. 11A shows exemplary lengths of a backward-oriented short constraining mechanism (BwS) and component thereof and a backward-oriented long constraining mechanism (BwL) and component thereof in accordance with one or more embodiments. FIG. 11B shows a front view of the exemplary backward-oriented constraining mechanism with horizontal loops, and FIG. 11C shows a top view of the exemplary backward-oriented constraining mechanism in accordance with one or more embodiments;

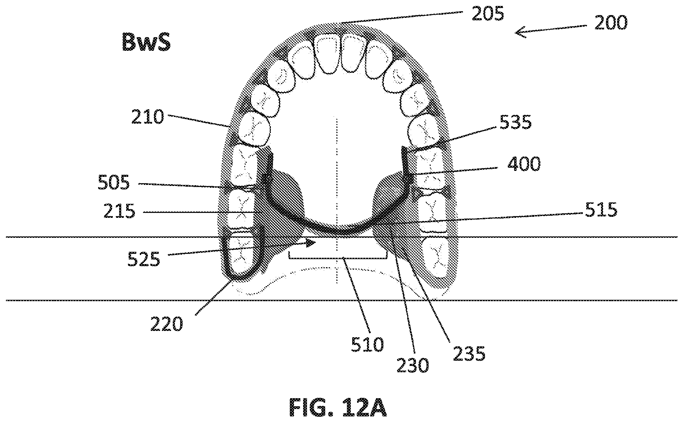

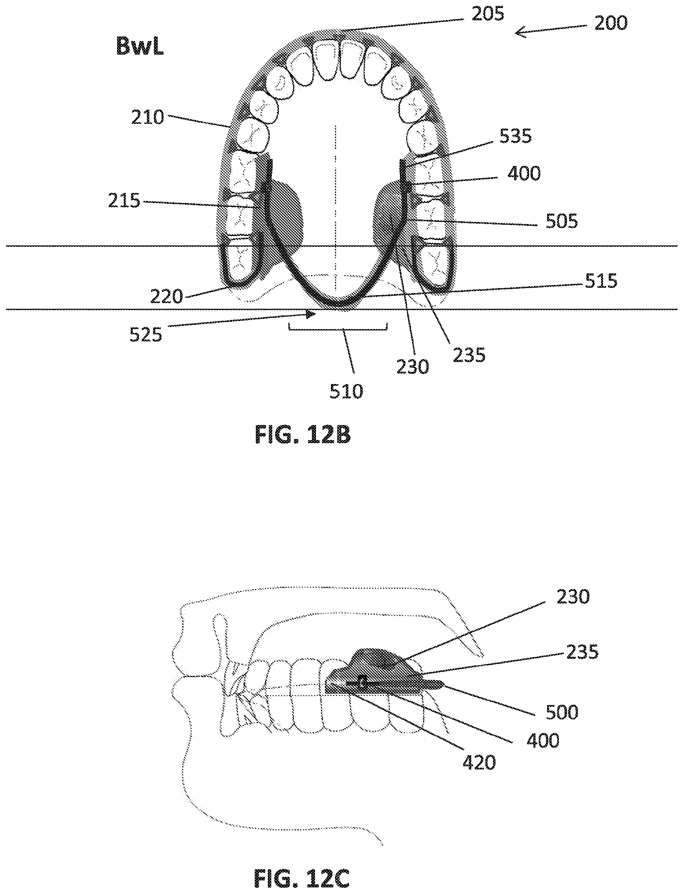

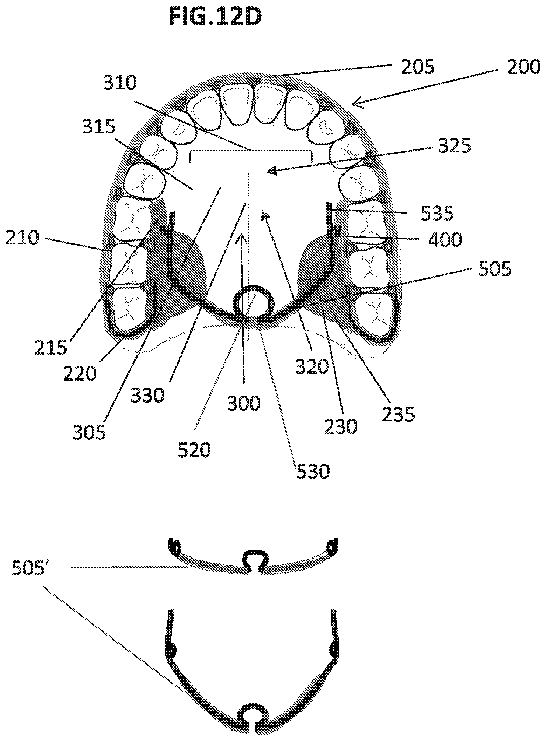

FIGS. 12A-D show an exemplary constraining mechanism having a backward orientation and its attachment to an attachment mechanism having wings via a fastening mechanism in accordance with one or more embodiments. FIG. 12A shows a bottom view in which the constraining mechanism is short and is in a posterior position, FIG. 12B shows a bottom view in which the constraining mechanism is long and is in a posterior position, and FIG. 12C shows a lingual side view in which the constraining mechanism is long and is in a posterior position; FIG. 12D illustrates a backward component 505 and 505' with a central buckle.

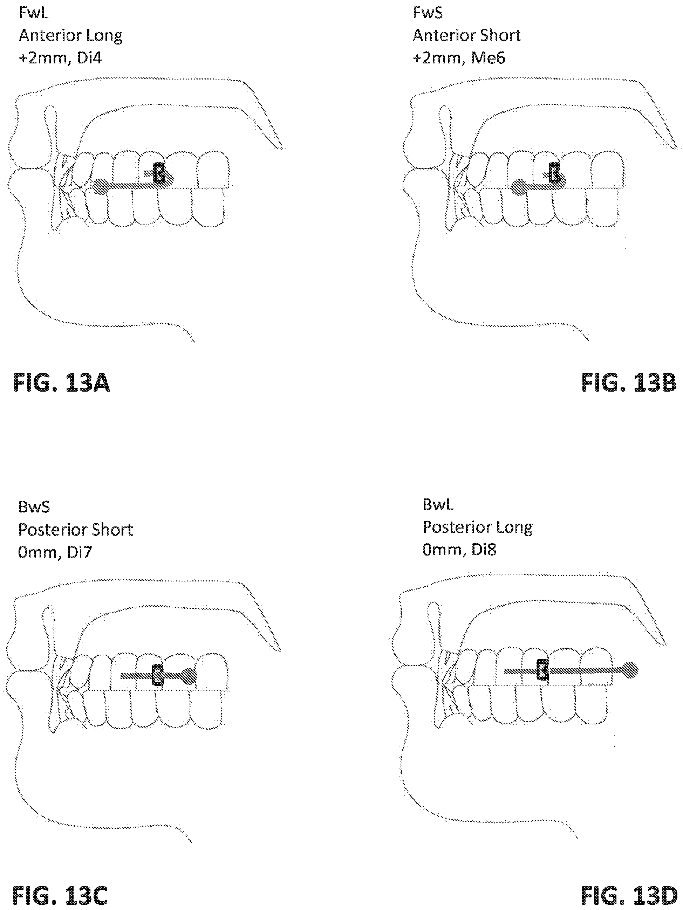

FIGS. 13A-D show the positioning of the component of exemplary constraining mechanisms to impose minimum (mild) constraint on the tongue in accordance with one or more embodiments. FIG. 13A shows the minimum constraint position on the dental arcade for a long, forward-oriented constraining mechanism (FwL), FIG. 13B shows the minimum constraint position for a short, forward-oriented constraining mechanism (FwS), FIG. 13C shows the minimum constraint position for a long, backward-oriented constraining mechanism (BwS), and FIG. 13D shows the minimum constraint position for a long, backward-oriented constraining mechanism (BwL);

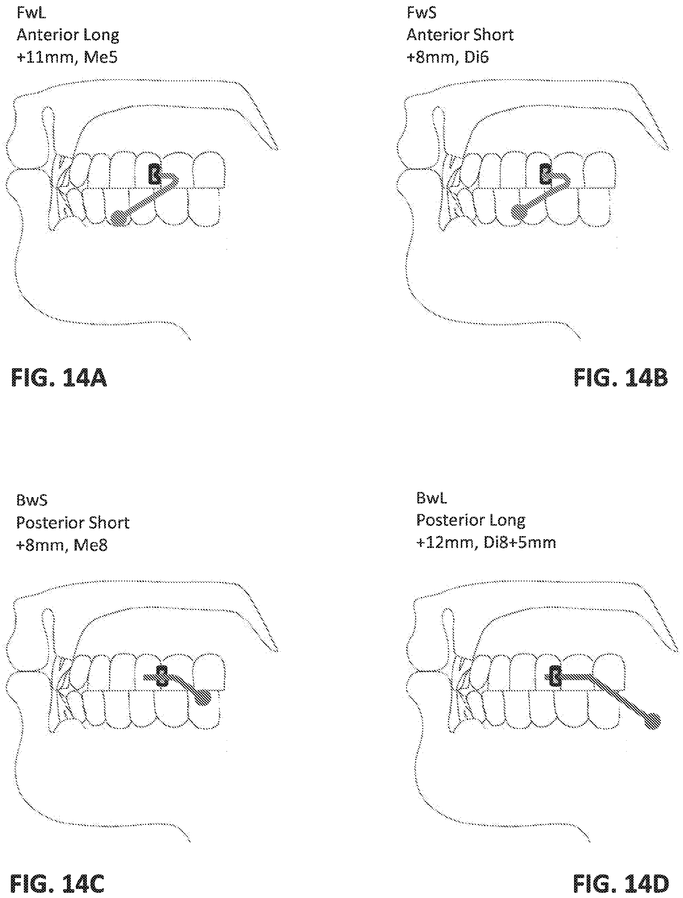

FIGS. 14A-D show the positioning of the component of exemplary constraining mechanisms to impose maximum (strong) constraint on the tongue in accordance with one or more embodiments. FIG. 14A shows the maximum constraint position on the dental arcade for a long, forward-oriented constraining mechanism (FwL), FIG. 14B shows the maximum constraint position for a short, forward-oriented constraining mechanism (FwS), FIG. 14C shows the maximum constraint position for a short, backward-oriented constraining mechanism (BwS), and FIG. 14D shows the maximum constraint position for a long, backward-oriented constraining mechanism (BwL);

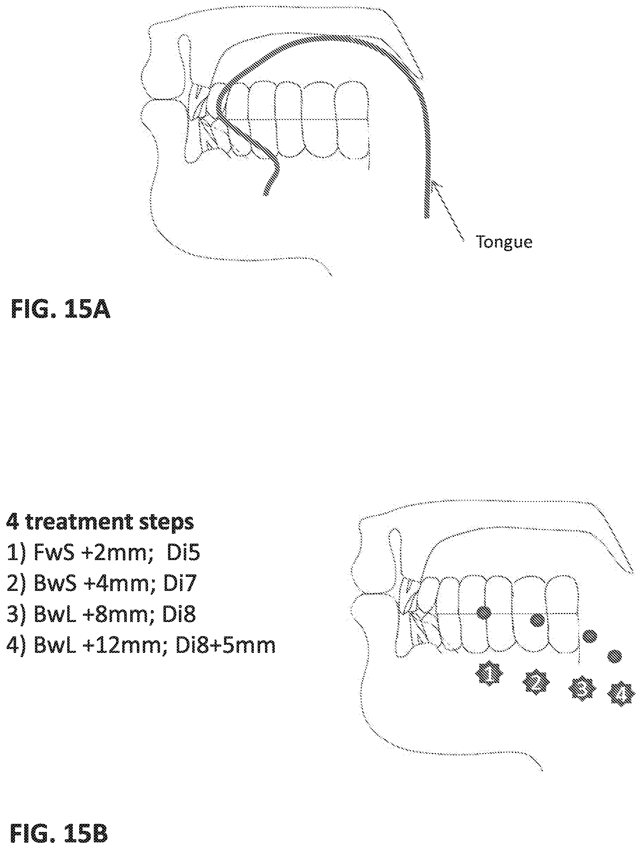

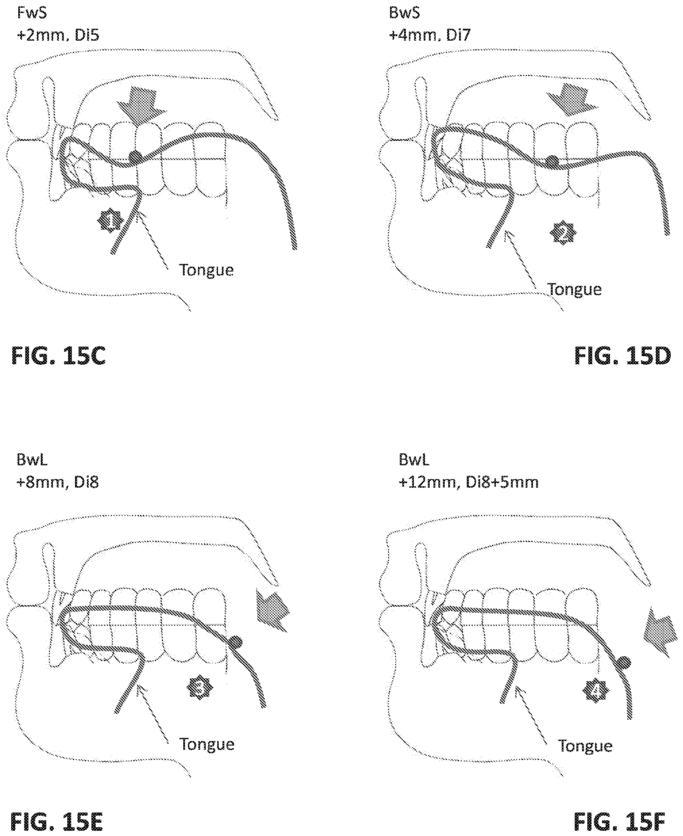

FIGS. 15A-F show an exemplary treatment plan for treating a tongue condition associated with obstructive sleep apnea (OSA), in accordance with one or more embodiments. FIG. 15A shows an exaggerated schematic of the shape of an abnormally positioned tongue associated with obstructive sleep apnea, and FIG. 15B shows a diagram of the locations of the apex of the component of the constraining mechanism for each of four exemplary steps (represented by the series of dots) for treating the tongue condition shown in FIG. 15A. FIGS. 15C-F show the type of component and the positioning of the apex of the component of the constraining mechanism relative to the tongue for the respective four treatment steps;

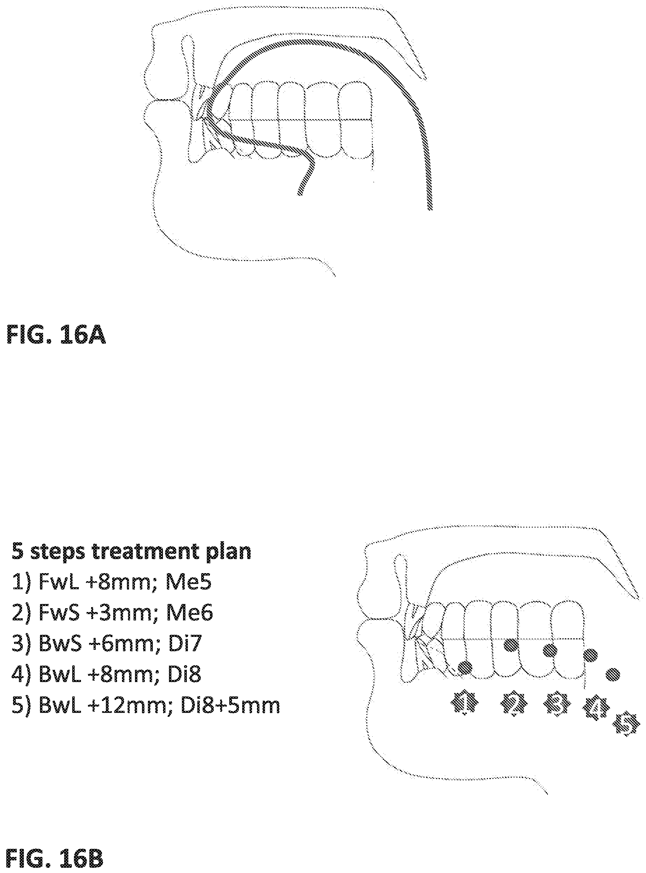

FIGS. 16A-G show an exemplary treatment plan for treating a tongue condition associated with malocclusion and/or snoring, in accordance with one or more embodiments.

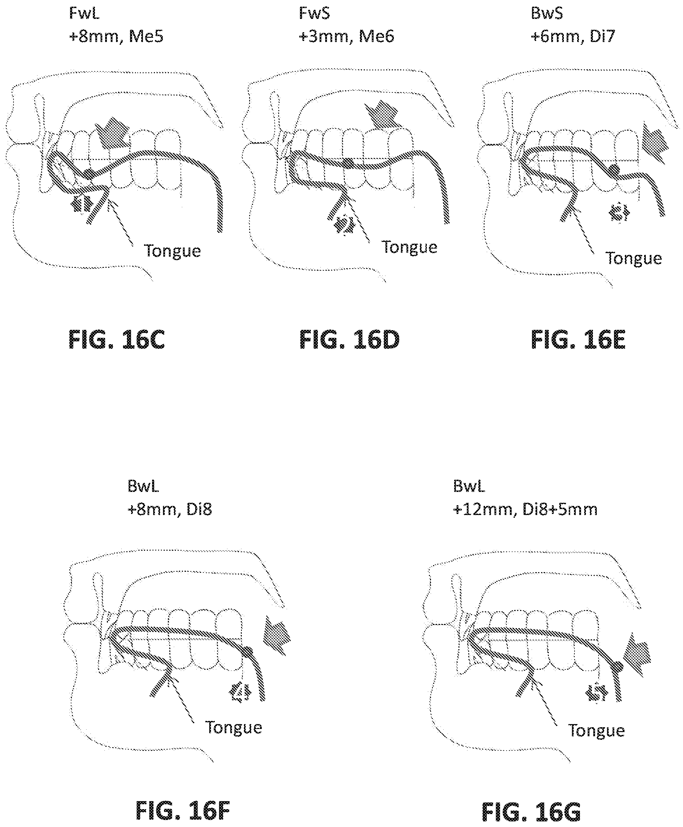

   FIG. 16A shows an exaggerated schematic of the shape of an abnormally positioned tongue associated with malocclusion and/or snoring, and FIG. 16B shows a diagram of the locations of the apex of the component of the constraining mechanism for each of five exemplary steps (represented by the series of dots) for treating the tongue condition shown in FIG. 16A. FIGS. 16C-G show the type of component and the positioning of the apex of the component of the constraining mechanism relative to the tongue for the respective five treatment steps;

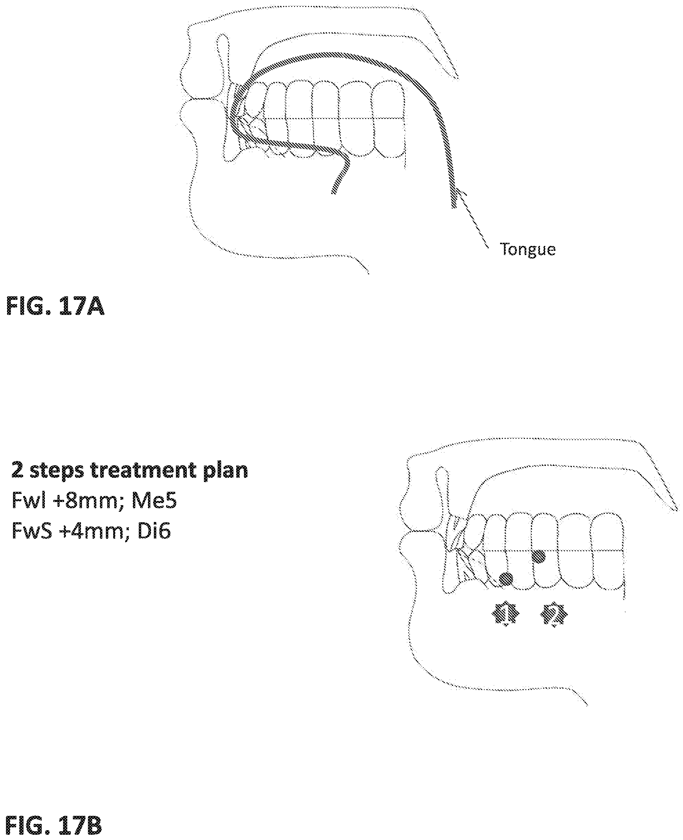

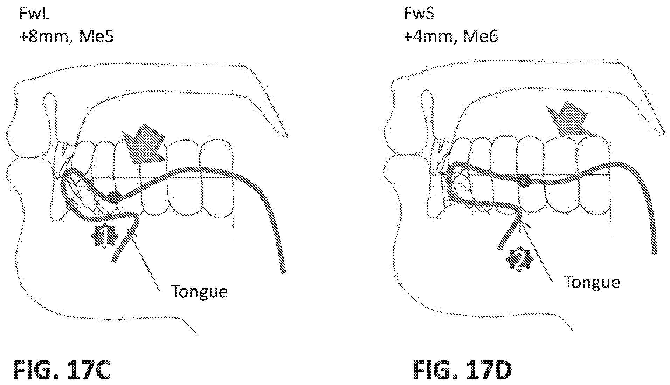

FIGS. 17A-D show an exemplary treatment plan for treating a tongue condition associated with malocclusion, in accordance with one or more embodiments. FIG. 17A shows an exaggerated schematic of the shape of an abnormally positioned tongue associated with malocclusion, and FIG. 17B shows a diagram of the locations of the apex of the component of the constraining mechanism for the two exemplary steps (represented by the series of dots) for treating the tongue condition shown in FIG. 17A. FIGS. 17C-D show the type of component and the positioning of the apex of the component of the constraining mechanism relative to the tongue for the respective two treatment steps; and

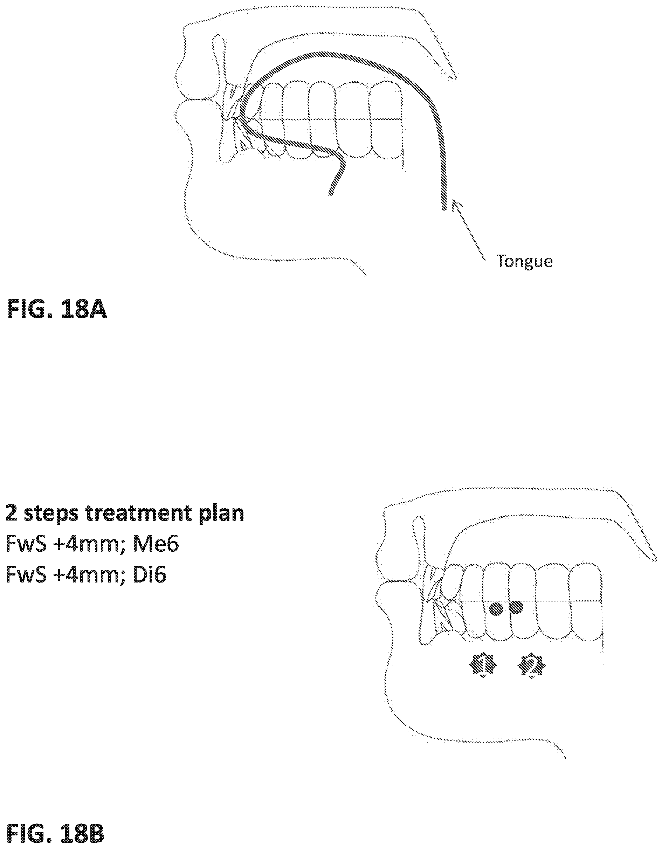

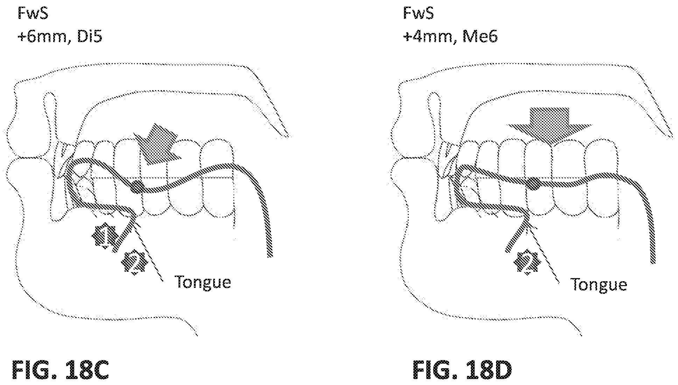

FIGS. 18A-D show an exemplary treatment plan for treating a tongue condition associated with persistent atypical swallowing, in accordance with one or more embodiments. FIG. 18A shows an exaggerated schematic of the shape of an abnormally positioned tongue associated with persistent atypical swallowing, and FIG. 18B shows a diagram of the locations of the apex of the component of the constraining mechanism for the two exemplary steps (represented by the series of dots) for treating the tongue condition shown in FIG. 18A. FIGS. 18C-D show the type of component and the positioning of the apex of the component of the constraining mechanism relative to the tongue for the respective two treatment steps.

### DETAILED DESCRIPTION OF CERTAIN EMBODIMENTS

By way of overview and introduction, apparatuses and methods for limiting the movement of the tongue are described herein. It can be appreciated that despite the currently practiced strategies used by others to reduce tongue dysfunction and the effects of its associated medical conditions, the treatments are oftentimes ineffective or unduly invasive or both.

In an effort to combat the effects of tongue dysfunction as well as macroglossia which has similar effects, the apparatuses and methods described herein limit the movement of a person's tongue and help to train or retrain the person's tongue such that the abnormal morphology of the tongue and the effects of tongue dysfunction are ameliorated. The apparatuses for limiting the movement of and retraining the tongue as described herein are dental appliances that can comprise an attachment mechanism for (preferably releasably) attaching the dental appliance onto selected teeth of a person.

The connector connecting the first and second surfaces of the attachment mechanism may but need not be made of the same resinous material and the first surface of the attachment mechanism. Indeed, it may but need not be simply an extension of the first surface wrapping around the last upper tooth and integrating with the corresponding second surface. As shown, the connector is not disposed between adjacent teeth. As described, the connector can be a resinous material or can be formed of another material, such as a metal wire, etc.

The constraining mechanism can include a component that spans between left and right surfaces of the attachment mechanism (and left and right sides of the dental arcade) and sits approximately at the occlusal plane of the person's mouth, thereby confining the range of motion of the tongue. The dental appliances as described herein can be shaped to conform to a person's unique anatomy and to the tongue's abnormal posture and resulting abnormal oral morphology and dysfunction, and can be selectively adjusted in the course of treatment to retrain the movements of a person's tongue, thereby improving their specific medical condition.

The following description is directed to dental appliances and methods for limiting and retraining the tongue. The referenced appliances and methods are now described more fully with reference to the accompanying drawings, in which one or more illustrated embodiments and/or arrangements of the apparatuses and methods are shown. The apparatuses and methods are not limited in any way to the illustrated embodiments and/or arrangements as the illustrated embodiments and/or arrangements described below are merely exemplary of the present apparatuses and methods, which can be embodied in various forms as appreciated by one skilled in the art. Therefore, it is to be understood that any structural and functional details disclosed herein are not to be interpreted as limiting the present application, but rather are provided as a representative embodiment and/or arrangement for teaching one skilled in the art one or more ways to implement the present apparatuses and/or methods. Moreover, just because a certain feature is depicted in combination with a particular set of other features, no intent to so limit the invention can be inferred and each feature can be combined with any other set of other features. Accordingly, certain aspects of the present apparatuses and methods can take the form of an entirely hardware embodiment or an embodiment combining software and hardware.

### Definitions

The following terms and phrases include the meanings provided below unless the context clearly indicates otherwise.

"Posterior zone" generally refers to the area of the mouth from the distal end of the first molar to the location where the tongue slopes steeply downward.

"Anterior zone" generally refers to the area of the mouth from the incisors to the mesial end of the first premolars.

"Central zone" generally refers to the area of the mouth from the mesial end of the first premolars to the distal end of the first molars.

"Lateral edges" generally refer to the left and right-side portions of the tongue.

"Tip" generally refers to the forward most portion of the tongue.

"Substantially circumferential" is defined, in at least one embodiment, as partially encircling the dental arcade of the upper (or lower) jaw, leaving the lingual side of at least the front teeth uncovered but extending to the lingual side of at least one rear tooth (and preferably two or three rear teeth).

"Dental arcade" generally refers to a set of upper (or lower) teeth of a person or animal.

The "rear corresponding tooth" generally refers to one or more teeth in the posterior zone of the mouth. In one embodiment, the rear corresponding tooth is a molar.

A "connector" generally refers to the part of the dental appliance that connects the first surface and the second surface of the dental appliance. In some embodiments, the connector at least partially encircles a rear area such as the terminal corresponding tooth or teeth.

The "occlusal plane" generally refers to an imaginary surface that theoretically touches the incisal edges of the incisors and the tips of the occluding surfaces of the posterior teeth. The occlusal plane represents the mean of the curvature of the surface.

A "three-dimensional space" refers generally to a volume.

"Tissue" can generally refer to teeth, gums, the palate, or the tongue; soft tissue" refers to palate, gums and tongue except when the context requires only gums and palate (for example, as in the description of wings).

"C-shaped" can generally refer to any curved or three-sided rectilinear, and can also include a "U" shape.

"Oral," used in reference to the tongue, includes both buccal (within the mouth) and pharyngeal (down towards the pharynx) portions of the tongue.

"Lateral expansion" with reference to the component means expanding the length of the component so that the appliance exerts lateral (i.e., transverse to the sagittal plane) pressure on the dental arcade and thereby contributes to similarly lateral (transverse) expansion of the palate.

### Description of Certain Embodiments

Various embodiments of the present application are described herein in detail with reference to the drawing figures, wherein like references numerals represent like parts and assemblies throughout the figure set. The present disclosure relates to one or more dental appliances and one or more methods related to the use of the dental appliances. The dental appliances are described in the context of the oral cavity (buccal cavity) of a person who would be wearing the appliance. In one or more embodiments, the one or more dental appliances can be used in one or more methods of treatment for various medical conditions related to oral and/or mandibular malfunctions.

FIGS. 1A-B show various aspects of the dental arcade of a person. FIG. 1A shows a side view of the left side of the top and bottom dental arcade, and FIG. 1B shows a bottom view of three teeth on the left side of the upper dental arcade of a person. As shown by the numbering in FIG. 1A, on each side of his or her mouth, a person generally has 8 teeth on each side of the top dental arcade and 8 teeth on each side of the bottom dental arcade. As such, the dental appliances of the present invention as described herein are described with reference to a person having 8 teeth on each side of the top dental arcade; however, it should be understood that the dental appliance can be fitted in persons having more or less than the normal number of teeth. As shown in FIG. 1B, each tooth is generally oriented in the dental arcade such that it has a "mesial side" (side towards the "anterior midline", i.e., the line between the front two teeth, defining the left and right side of the dental arcade), a "distal side" (side toward the most posterior tooth) a "lingual side" (side adjacent to the tongue) (or a "palatal side") and a "vestibular side" (side adjacent to the inside of the cheek or lips). When used in connection with the appliance, the terms "left" and "right" will denote the side of the appliance when it is worn, which would then correspond to the left and right side of the wearer's dental arcade and more generally mouth and oral cavity.

### Attachment Mechanism

In accordance with one or more embodiments, the dental appliances of the present application include an attachment mechanism that attaches the dental appliance onto the top dental arcade within the mouth of a person. In one or more embodiments, the attachment mechanism is configured to snap onto the top dental arcade of the person to releasably secure the dental appliance to the dental arcade. The attachment mechanism, however, does not prevent upper to lower dental arcade contact.

The attachment mechanism can include a substantially but not fully circumferential band for sitting on or about the dental arcade of a person (for example, when the appliance is worn, the attachment mechanism may cover all part or all of vestibular exposed (crown) surface of the upper teeth vestibular surfaces. While it may expend slightly over the gum, this is not preferred as it tends to be uncomfortable for the patient. The attachment mechanism preferably does not cover the occlusal (biting) surfaces of the teeth, permitting maximal intercuspation occlusion and contributing to a more natural cutting, shredding and chewing function. However, embodiments covering the biting surfaces too (and optionally also the entire lingual surfaces of the upper teeth) can be envisioned and are within the scope of the present invention. Regarding the lingual side, in some preferred embodiments, the second surfaces of the attachment mechanism extend over a limited number of rear teeth, whether the dorsal section of the component faces backward or forward.

The attachment mechanism attaches to the dental arcade and is releasably held there by pressure, slightly pinching the molars used for attachment. The attachment mechanism can be easily released by the patient by application of pressure and is easy to wear and take off, leaving the lingual surfaces of most of the patient's teeth uncovered. This contributes to the patient experiencing a more natural feeling. The dental appliances described herein can also include a constraining mechanism removably connected to the attachment mechanism. The teeth used for attachment are preferably at least one rear tooth on each side of the upper dental arcade of the patient. Two teeth or even three teeth on each side are typical. But an objective of some embodiments is that the lingual surfaces of the remaining teeth and notably the front teeth should remain uncovered. Similarly, as stated above, the lingual surfaces of a substantial majority of the upper teeth are not covered leaving the lingual surfaces of the teeth substantially exposed. This has several advantages: the tongue can enjoy contact with the incisor papilla which contributes to physiological proprioception; the appliance is more flexible, more comfortable and easier to wear and take off; less resinous material is required for manufacturing the appliance; the lingual surfaces of the upper teeth (other than the attachment teeth) remain exposed to the tongue, which gives the wearer less of a "foreign object in the mouth" feeling; and essentially full contact between the upper and lower teeth is permitted, leaving the chewing, shredding and cutting function uninhibited and contributing to the natural feeling. This is to be contrasted with the bleaching retainers, mandibular advancement splints, or modern orthodontic aligners or retainers currently on the market, such as InvisalignTM which cover the entire tooth surface.

Various aspects of the attachment mechanism, in accordance with one or more embodiments, are shown in FIGS. 2A-5B.

FIG. 2A shows a bottom view of an attachment mechanism 200 of a dental appliance on the top dental arcade in accordance with one or more embodiments. Referring to FIG. 2A, the attachment mechanism 200 comprises a substantially circumferential band ("circumferential band") 205. In one or more embodiments, the circumferential band 205 comprises a resinous material (e.g., a polymeric material, such as an acrylic resin band, preferably translucent or transparent) that is in contact with the top dental arcade when the dental appliance is worn. In one or more embodiments, the entire circumferential band 205 is made of a resinous material. In at least one embodiment, one or more portions of the circumferential band 205 are made of a resinous material. In one or more implementations, the substantially circumferential band 205 is in contact with the vestibular side of the top dental arcade. The band serves to releasably secure the appliance to the dental arcade.

In general, the material for the attachment mechanism and in particular the band surfaces, and optionally also connector, is any physiologically acceptable, preferably medical grade (with optional antibacterial additive), resinous material that is solid in the temperature range of its use (for example with a melting point over about 125 degrees C), having a bulk density roughly the same as water, a tensile strength of the order of about 50 to about 80 and preferably about 60 Mpa with a Young modulus over 2 Gpa, and preferably between 2 and 3 Gpa, a Knoop hardness of about 18 to about 20, good optical properties (preferably bright and transparent with color being an option), sufficient elasticity to be put on and removed by the exercise of simple pressure of the hand, and good resistance to oxidation and more generally good aging characteristics so that it can remain in the environment of the mouth (exposed to air, food and oral fluids) for at least 18 months and preferably for 2 years essentially without losing mechanical properties.

The circumferential band 205 includes a first surface 210 that is adapted to be seated on the vestibular side of the top dental arcade when the appliance is worn and preferably to generally conform to the vestibular surface of the tooth crowns. The circumferential band 205 also includes a pair of second surfaces 215 that when worn will be located along the lingual side of at least one rear tooth (e.g., a rear molar tooth) on each side of the top dental arcade. In other words, the circumferential band 205 comprises a left second surface to be located on the lingual side of at least one rear tooth on the left side of the top dental arcade, and a right second surface to be located on the lingual side of at least one rear tooth on the right side of the top dental arcade. In the embodiment shown in FIG. 2A, the pair of second surfaces 215 are adapted to be located along the lingual side of the three rearmost teeth on each side of the top dental arcade. The second surfaces 215 of the attachment mechanism do not hinder all tongue-to-teeth contact at the location of the second surfaces 215. As stated above, typically, each of said first and second surfaces span one, two or three rearmost teeth. Longer surfaces may be employed as long as they contribute to the attachment function and are not uncomfortable. But it is desirable to preserve tongue-to teeth contact, so limiting the number of teeth which are spanned by the first and second surfaces by limiting the length of these surfaces without compromising the attachment function. The latter includes snug fitting which is releasable by the exercise of slight pressure by the wearer's hand.

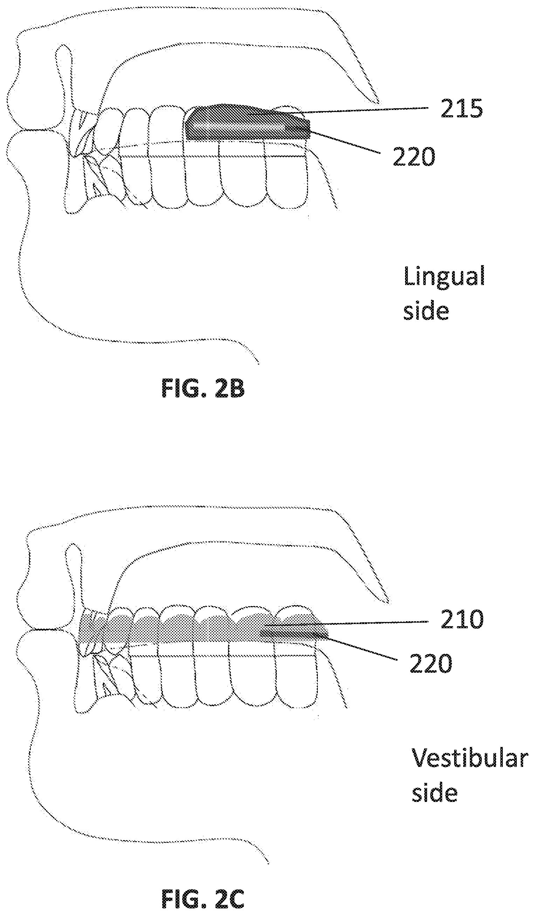

It will be appreciated that the circumferential band 205 can be in the form of a plurality of discontinuous resinous sections or portions. More specifically and according to one embodiment, the circumferential band 205 can be formed of a first resinous portion that extends along the first surface 210, a second resinous portion that extends along one of the second surfaces 215 and a third resinous portion that extends along the other of the second surfaces. It will be appreciated that by segmenting the second and third resinous portions, there is a gap therebetween along the second surface 215 in that a curved front portion of the first resinous portion lacks a corresponding resinous portion along the second surface that faces this first surface. By segmenting the resinous portion along the second surface 215 into two discontinuous segments, the rigidity of the device is reduced since if the resinous portion extended along the entire second surface, the dental appliance would have a rigidity that would not be desired for many applications. There is a space between the first resinous portion and the second resinous portion for receiving teeth and a space between the first resinous portion and the third resinous portion for receiving other teeth (see, Fig. 2C showing teeth protruding above the resinous portion). Teeth thus can be fit between the resinous portions. The three resinous portions are thus not in direct contact with one another but instead are coupled to one another using a connector as described below. On the other hand, there can be an integrated, one-piece, circumferential band, as shown, e.g., in FIG. 12A, with the connector being the portion of the band connecting the vestibular surface to a lingual, left or right, surface.

With continued reference to FIG. 2A, the circumferential band 205 can also include a connector 220 that joins the first surface 210 and the second surface 215. In one or more embodiments, the connector 220 joins or connects the first surface 210 and the second surface 215 at the rearmost tooth on each side of the top dental arcade. In one or more embodiments, the connector 220 can extend from the middle of the distal side of the rearmost tooth to the entire length of the vestibular and lingual sides of the rearmost at least one tooth of the top dental arcade. In at least one embodiment, the connector 220 can further extend to the entire length of the vestibular and/or lingual sides of the second or also third rearmost tooth on each side of the top dental arcade. In one or more embodiments, the connector 220 can be a wire (e.g., stainless steel wire, preferably medical grade steel, or a specialty steel alloy such as Elgiloy^{™} (Co-Cr-Ni Alloy made by Elgiloy Specialty metals, Elgin, Illinois). In some embodiments, the wire that can be in the form of a continuous wire that joins the first surface 210 to the second surface 215 or can be in the form of segmented wires that each joins the first surface to one second surface 215. In certain embodiments, the wire can be embedded within the resinous material of the circumferential band. The wire can be positioned within the resinous material of the circumferential band such that it sits substantially adjacent to the middle of each tooth that it surrounds.

In the above-described embodiment, one connector 220 joins the first resinous portion to the second resinous portion and one connector 220 joins the first resinous portion to the third resinous portion.

In one or more implementations, the circumferential band 205 is approximately 2 to 4 mm high so as not to cover the whole exposed vestibular surface of the teeth of the top dental arcade, and preferably to remain under the gum line. In at least one implementation, the circumferential band 205 can be approximately 1 to 2 mm high for patients with a smaller than normal dental arcade or approximately 4 to 6 mm high for patients with a larger than normal dental arcade. In one or more embodiments, the attachment mechanism 200, including the circumferential band 205, can be custom made to fit the measurement of a particular individual's top dental arcade. Accordingly, the attachment mechanism 200 can be specifically sized and shaped to snap onto the top dental arcade of the person to releasably secure the dental appliance on the dental arcade.

FIGS. 2B-C show side views of the attachment mechanism in accordance with at least one embodiment. In particular, FIG. 2B shows a side view from inside the dental arcade, showing the lingual side of the attachment mechanism, featuring the second surface 215 and the connector 220 attached to the second surface 215. FIG. 2C shows a side view from the outside of the dental arcade, showing the vestibular side of the attachment mechanism featuring the first surface 210 and the embedded connector 220.

In at least one embodiment, the attachment mechanism 200 can include an arcuate support member that follows the contour of the person's palatal surface (i.e., roof of the mouth). The arcuate support member is disposed between the pair of second surfaces and connected to the lingual side of the second surfaces. FIG. 3A displays a bottom view of an embodiment of the attachment mechanism 200 of the dental appliance having an arcuate support member, in accordance with one or more embodiments. As shown in FIG. 3A, the attachment mechanism 200 includes an arcuate support member 225 that spans between and attaches to the second surfaces 215. In one or more implementations, the arcuate support member 225 arches upward following the contour of the person's palate, but preferably does not touch the palate to avoid discomfort to the user. The arcuate support member provides increased rigidity, strength, and support for the dental appliance. In one or more implementations and as shown in FIG. 3A, the arcuate support member 225 can be an arch made of resin. In certain embodiments, the length of the arcuate support member can approximately equal the length of the second surfaces, and in at least one embodiment (as shown in FIG. 3A) the width of the arcuate support member 225 can equal a portion of the length of the second surfaces 215 in that in this particular embodiment the width of the arcuate support member 225 does not extend the entire length of the second surface 215 (it might do so however if the number of teeth spanned by surfaces 215 is reduced).

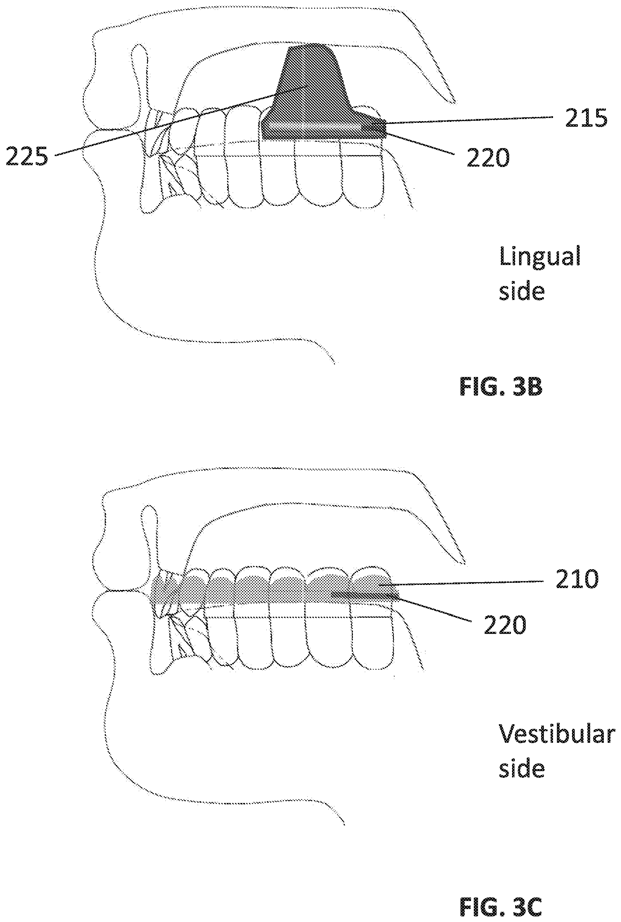

FIGS. 3B-C show side views of the attachment mechanism having the arcuate support member 225 in accordance with at least one embodiment. In particular, FIG. 3B shows a side view from inside the dental arcade, showing the lingual side of the attachment mechanism 200, featuring the arcuate support member 225 attached to the second surface 215. This view also shows the connector 220 attached to the second surface 215. FIG. 3C shows a side view from the outside of the dental arcade, showing the vestibular side of the attachment mechanism, featuring the first surface 210 and the integrated connector 220, but the arcuate support member is not visible (in any event, it is optional).

In at least one embodiment, the circumferential band 205 can include a wing attached to the lingual side of one or both second surfaces. The wing can be configured to hold one or more sensing devices (sensors) as described below. In one or more implementations, the wing can be made of a resinous material like the rest of the circumferential band, and the sensor can be embedded within the resinous material of the wing or otherwise attached to the wing. In at least one embodiment, the wing is not in contact with the palate or the tongue as, when the appliance is worn, the tongue is under constraint. As such, embedding the sensor within the wing allows for the sensor to operate without touching the tissue (e.g., palate, tongue) of the person wearing the dental appliance. Fig. 5A shows one exemplary wing structure. Two wings can be included as shown, for symmetry and proprioception, or to accommodate a greater number of sensors than can be borne by a single wing, if such plurality of sensors should be desirable.

The one or more sensors can be used for monitoring various aspects of the mouth while the dental appliance is worn, including temperature, pH and/or sound (the sound sensor would evaluate snoring; the pH sensor would measure pH to evaluate for example mouth breathing). For example, a temperature sensor can be included in the wing of the circumferential band to periodically measure the temperature at or near the dental appliance. A temperature sensor can be used to monitor a person's compliance in using the dental appliance regularly. For example, if the temperature sensor determines that the temperature at or near the device is in the range of approximately 35 to 38°C (95 to 100.4°F), then the device is currently being worn by the user. However, if the temperature readings of the sensor are well below that temperature range, for example at 20 or 22°C, the device is likely not currently being worn by the user. The temperature sensor can also be configured to evaluate the mouth breathing versus nasal breathing of the user. For example, if the temperature sensor determines that the temperature at or near the device is in the range of approximately 35 to 38°C (95 to 100.4°F), then the wearer of the dental appliance is primarily nasal breathing. On the other hand, if the temperature sensor determines that the temperature at or near the device is in the range of approximately 30 to 35°C (86 to 95°F), then the wearer of the appliance is likely mouth breathing. To ensure accurate temperature readings by the temperature sensor, the sensor is not in contact with the tongue or gums of the user so that the temperature readings are not impacted by the tissue of the user. An exemplary sensing device (sensor) 230 is shown at FIG. 4, in accordance with at least one embodiment.

The sensor can vary in size and shape depending on the size of the wing it is attached to on the dental appliance. In embodiments where an arcuate support is provided, one or more sensors can be embedded in the arcuate support. For instance, in an exemplary embodiment, the sensor can be 9 x 13 mm in size. The sensor can include various features, including but not limited to a processor, a memory, an antenna, and/or a battery. In one or more implementations, the sensor can be configured to communicate wirelessly with one or more remote computing devices. For example, readings gathered by the sensor can be transferred to one or more remote computing devices via a radio frequency identification (RFID) tag attached to the sensory device. The one or more sensors of the present dental appliance can include hardware and/or software components that can be commissioned on a commercial basis.

For example, miniature wireless sensors susceptible to an inductive battery charge are commercially available and have been described for example in Arbinger FX et al, a publication of the Fraunhofer Institute titled Wireless Charger Chip for Smart Card Applications (https://www.iis.fraunhofer.de/content/dam/iis/de/doc/lv/.../wireless_battery_charger.pdf last visited July 1, 2017).

The sensor apparatus comprises the following components, each performing the following function or functions.: a housing; an impermeabilized microprocessor module for processing and memory (MCU) for data input, processing and storage; a temperature sensor for obtaining temperature readings (and/or a sound or pH sensor for other relevant data generation, optionally more than one such sensing device); a communication module including an RFID and antenna (provided with a link at an appropriate frequency such as 13.56 MHz); a charging module and a rechargeable battery; and an optional on/off switch. The foregoing components are either commercially available or their design and construction are within the skill of the art. It will be appreciated that this type of sensor can be incorporated into the devices of the present invention.

FIGS. 5A-B show an exemplary attachment mechanism featuring two wings and a sensor in accordance with at least one embodiment. In particular, FIG. 5A displays a top view of an attachment mechanism 200 featuring a wing 235 attached to each second surface 215, and a sensor 230 embedded within the wing 235 of the right second surface. In one or more embodiments, and as shown in FIG. 5A, the wing 235 is made of resinous material and the resinous wings are fused to the resin of their respective second surfaces 215. In one or more implementations, the wings 235, like the rest of the attachment mechanism 200, can be individually sized and shaped to contain a sensor. If there is no sensor the wing is not needed; in any event, the sensor is optional. There two wings can be used for additional sensing functions or simply for symmetry and proprioception. In an exemplary implementation, the wing can be approximately 13 - 15 mm long at the base and about 8 - 9 mm long at the top, and approximately 3 mm thick. Its base is attached (e.g., fused) to the second surface while the wing extends obliquely and may taper upwards of the second surface so as not to interfere with the tongue or touch the palate or teeth. FIG. 5B shows a side view of the second surface of the attachment mechanism of FIG. 5A, showing the lingual side of the mechanism, featuring the sensor 230 embedded within the wing of the right second surface 235.

### Constraining Mechanism

The dental appliances of the present application also include a constraining mechanism that is preferably removably secured to the attachment mechanism via a fastening mechanism. In one or more embodiments, a pair of wired ends of the constraining mechanism is responsible for securing the constraining mechanism to the left and right second surfaces of the attachment mechanism and making it possible for the constraining mechanism to hold in place against pressure by the tongue. The constraining mechanism includes a component (e.g., a wire) that spans between the pair of second surfaces 215 of the attachment mechanism and is located approximately at the occlusal plane of the mouth of the wearer. The positioning of the component approximately at the occlusal plane confines the range of motion and the movement of at least part of the tongue. In general, the constraining mechanism forces the tongue of the user to flatten over time, as well as tip slightly upward such that the apex of the tongue (tip of the tongue) sits at the upper incisor papilla when the tongue is in a resting position. Using a series of different components for the constraining mechanism and/or adjustments to the positioning of the constraining mechanism, the tongue is progressively drawn forward and/or downward. During this process of flattening the tongue, when the tongue is pushing on the constraining mechanism, part of the upward force will convert into a light horizontal, externally-oriented force by the constraining mechanism, which can result in (necessary) lateral or transverse palate enlargement which helps render the palate more shallow. This externally-oriented force by the constraining mechanism is in addition to a transverse force exerted by the sides of the tongue on the molars when the device is worn by the user.

The component can be rearward-oriented (backward-oriented) or forward-oriented depending on the treatment plan for the type of tongue-related condition to be corrected. In particular, when the component has a forward orientation, the component is centrally positioned relative to the second surfaces and a forward portion of the circumferential band. Further, the forward-oriented component is seated at a center portion of tongue at approximately the occlusal plane of the wearer. When the appliance is worn, the forward-oriented component is configured to limit movement of a central zone of the tongue and thereby confine the range of motion of the tongue to a more limited three-dimensional space relative to the space the tongue generally moves within. While the forward-oriented component limits the movement of the central zone of the tongue, it still allows for the tip and lateral edges of the tongue to perform movements necessary for speech and swallowing. The forward-oriented component spans between and is adjacent to at least a portion of the left and right second surfaces.

Conversely, a component having a rearward orientation is configured to limit movement of the posterior zone of the tongue and thereby confine the range of motion of the tongue of the wearer to a more limited three-dimensional space relative to the space the tongue generally moves in the absence of the dental appliance. While the rearward-oriented component limits the movement of the posterior zone of the tongue, it still allows for at least the anterior and the central zone and the lateral edges of the tongue to perform movements necessary for speech and swallowing. The rearward-oriented component spans between and is secured to the left and right second surfaces at a rear portion of the dental appliance.

The component, whether forwardly or backwardly oriented, is generally a metallic wire. The metallic wire can be made of one or more metals. For example, the metallic wire can be made of stainless steel, titanium molybdenum alloy, Bendalloy^{®}, or Elgiloy^{®}. In an exemplary embodiment, the wire can be 0.9 mm in diameter. However, in other embodiments, the diameter of the wire can be of a different diameter depending on the anatomy and/or condition of the user and the required flexibility of the component. In one or more embodiments, at least a portion of the metallic wire is coated with a resinous material (e.g., acrylic resin), which provides improved comfort and tolerance of the dental appliance for the user, thereby leading to increased compliance by the user. In one or more embodiments, the component, regardless of orientation, is generally C-shaped with a dorsal section of the component disposed transversely to a longitudinal axis of the appliance. The dorsal section of the component is chiefly responsible for limiting the movement of the tongue.

The component is generally more flexible at the dorsal section to adapt to the change in width of the dental arcade over the time the dental appliance is worn. In contrast, the ends of the constraining mechanism are generally more rigid as compared with the dorsal section, as the ends are designed to resist strong upward forces exerted by the tongue.

It should be noted that the positioning as well as the length and size of the component of the constraining mechanism in the mouth of the user, and in particular the positioning of the component directing action on the tongue is to be determined by taking into account the person's oral morphology and dysfunction. Therefore, absolute dimensions of the constraining mechanism (and the dental appliance in general) in mm as discussed throughout are exemplary, and can be modified to adjust to a specific patient's oral morphology.

### Forward-oriented Constraining Mechanism

An exemplary constraining mechanism having a forward orientation is shown at FIGS. 6A-6C. FIG. 6A shows the length, in mm, of exemplary forward-oriented constraining mechanisms. In particular, FIG. 6A shows the length of a forward-oriented short constraining mechanism (FwS) and a forward-oriented long constraining mechanism (FwL) in accordance with one or more embodiments.

FIG. 6B shows a front view of an exemplary forward-oriented constraining mechanism, and FIG. 6C shows a bottom view of an exemplary forward-oriented constraining mechanism. As shown in FIGS. 6B-C, the constraining mechanism 300 includes a component 305 with a forward orientation. In this embodiment, the component 305 is C-shaped and comprises a dorsal section 310. Further, in this embodiment, the component 305 comprises a wire in which a portion of the wire (e.g., most of the dorsal section 310) is covered by a coating resin (e.g., polymeric material) 315. In one or more embodiments, the resin coating 315 is approximately 1-2 mm thick around the wire.

In one or more embodiments, and as shown in FIGS. 6B-C, the dorsal section 310 can comprise a central buckle 320 that allows for flexibility and optional lateral expansion of the component within the mouth of the user. The dorsal section 310 also features an apex 325, which in this embodiment is the open end of the central buckle 320. The central buckle 320 also comprises a closed end 330, such that the closed end 330 is oriented in the opposite direction (i.e., rearward orientation) relative to the dorsal section 310. In other words, in the embodiment shown at FIGS. 6B-C, the central buckle 320 comprises a rearward oriented arch attached centrally to or forming part of the central portion of the dorsal section 310 of the component 305. The apex 325 of the component is the point of maximum confinement of the tongue. As shown in FIGS. 6B-C, the coating resin 315 covers most of the dorsal section 310 but not the central buckle 320, which allows for increased width flexibility. The central buckle 320 also ensures lateral flexibility of the component, as it is angled upwards at an angle of 30 - 50° with respect to the plane of the component 305 and pointing away from the wearer's incisors. Accordingly, when the dental appliance is in use, the central buckle 320 is not in contact with palate of the user or the tongue of the user when the tongue is at its resting spot. The constraining mechanism 300 also comprises a pair of wired ends 335 configured to removably connect the constraining mechanism 300 with the attachment mechanism 200 as discussed in further detail below. The central buckle can be featured in the rearward-oriented component but the geometry will be reversed, the opening of the central buckle will be towards the back of the wearer's mouth and the angle of the component may need to be adjusted. This is illustrated in FIG. 12D. Component 505 is facing rearward. In this embodiment, a central buckle 520 is provided with its closed end 530. "Loose" component 505' in FIG. 12D is shown to illustrate a shorter rearward component comprising a central buckle.

In at least one alternative embodiment, the constraining mechanism 300 can also feature a pair of vertical (or oblique) loops (stops) (not shown), one on each side of the component 305. The pair of oblique or vertical loops are shown to be part of the component (e.g., a wire) but they are a feature of the adjusting mechanism and are located at an end section of the constraining mechanism proximate to the second surfaces of the attachment mechanism. The vertical loops allow for additional vertical flexibility in that the component 305 and the central buckle 320 can be adjusted up or down 30° (from about 0° to about 30° from the occlusal plane (or from the plane defined by the ends 335 of component 305. As such, in this embodiment, the oblique or vertical loops serve as a part of the adjustment mechanism. In one or more implementations, the oblique or vertical loops can include a thin (e.g., 1 mm) silicone coating around the loops. Vertical or oblique loops are illustrated in FIG. 11A-C in connection with the rearward facing component but the same could be included in the corresponding position of a forward component. In the absence of vertical loops vertical adjustment can occur by bending the end of the component to alter the angle the component presents to the basal plane defined by these ends.

The constraining mechanism 300, as exemplified in FIGS. 6A-C, is designed to be flexible to accommodate lateral (i.e., transverse to the sagittal plane) expansion of the dental arcade as a result of wearing the dental appliance. Specifically, when the palate of the user is too narrow, the pressure exerted by the tongue, on assuming a flatter and closer to proper shape/posture under pressure from component 305, can cause the dental arcade to enlarge over time as the tongue is retrained to assume and return to a closer-to-normal posture. More specifically, when the device is worn, the dental arcade can widen as the tongue flattens because resistance against the tongue from the component can cause the tongue to exert a lateral force on the lingual side of the molar teeth. Accordingly, the component 305 (e.g., wire) must be flexible enough in the transverse direction such that the expansion of the dental arcade by the tongue is not hindered. As such, the lateral flexibility of the wire of the component imparted by opening the central buckle allows for lateral enlargement of the dental arcade without the component directly exerting either a restraining or an expanding lateral force on the molar teeth. In one or more implementations, the constraining mechanism 300 having a component 305 with a forward orientation can be adjusted laterally by up to approximately 5 mm, by slightly opening the central buckle 320 if one is provided. This can be done by an orthodontist or other dental professional. Optionally, the buckle can be encased (e.g., by dipping) in a "bead" or coating of resinous material (not shown in Figs 6 or 7 but shown in FIGS. 8A-C in the absence of a buckle, which adequately illustrates the principle) for added comfort. The bead/coating can be removed before adjustment and a new coating can be applied.

In one or more implementations, when tongue dysfunctions persist, the constraining mechanism can be adjusted downward up to 35° (measured from the center or apex of the component relative to the plane defined by the ends of the component) to increase constraint on the tongue. This adjustment can be done manually by a dental health professional, using for example oblique or vertical loops such as 340 (or 540 for a rearward component) or, in the absence of loops by altering the angle between the ends of the component, e.g., 335, and the C-shaped portion of the component 305.

FIGS. 7A-C shows an exemplary dental appliance, including the constraining mechanism 300 having a forward orientation, in accordance with one or more embodiments. As shown in FIGS. 7A-C, the constraining mechanism 300 is removably secured to the attachment mechanism via fastening mechanism 400. Further, the constraining mechanism 300 is positioned centrally relative to the pair of second surfaces 215 of the circumferential band 205, and, more specifically, the component 305 of the constraining mechanism 300 spans between the pair of second surfaces 215. The component 305 is configured to sit at approximately the occlusal plane of the wearer or at an angle thereto. Additionally, the apex 325 of the dorsal section 310 sits at a central portion of the tongue. The constraining mechanism 300 having a forward orientation is configured to limit movement of the central portion of the tongue, thereby confining the range of motion of the tongue to a limited three-dimensional space and forcing the tongue to lie flat. In the meantime, while the dental appliance of is worn, the constraining mechanism 300 still allows for the tip and lateral edges of the tongue to perform movements necessary for speech and swallowing.

As mentioned above, FIGS. 7A-C show the fastening mechanism 400 of the dental appliance and its positioning and affixation on one hand to the attachment mechanism 200 and on the other hand to the forward oriented constraining mechanism 300, in accordance with at least one embodiment. In the embodiment of FIGS. 7A-C, the attachment mechanism includes one or two wings 235 fused to the resin of the respective second surfaces 215, and a sensor 230 embedded in one of the wings 235. The wing bearing no sensor has no function other than to create an impression of symmetry in the wearer. It can be omitted. The sensor bearing wing can be omitted altogether if no sensor is used. As shown in FIGS. 7A-C, the fastening mechanism 400 can be located on the lingual side of the second surfaces 215 of the attachment mechanism, spanning one or more of the three molars.

FIG. 7A shows the forward-oriented constraining mechanism 300 attached to the attachment mechanism 200 via the fastening mechanism 400 at an anterior position, such that the apex of the dorsal section 310 (here, the central buckle 320) is located adjacent to the second premolar and adapted to be substantially anterior to the three molars. FIG. 7B shows the forward-oriented constraining mechanism 300 attached to the attachment mechanism 200 via the fastening mechanism 400 at a more posterior position relative to the positioning in FIG. 7A such that the apex of the dorsal section 310 (e.g., the central buckle 320) is adapted to be located adjacent to the first molar and substantially posterior to the premolars. Accordingly, as shown by FIGS. 7A and 7B, the position of the constraining mechanism 300 in the mouth of the user can be adjusted by changing along the wired ends 335 and is (preferably removably) secured in the desired position by the fastening mechanism. In FIG. 7A, the wired end 335 is fastened by the fastening mechanism 400 at a location proximate to the end of the dorsal section. In contrast, in FIG. 7B, the wired end 335 is fastened by the fastening mechanism 400 at or near the distal end of the wire end 335. In one or more implementations, the constraining mechanism can be adjusted between an anterior position (FIG. 7A) and a posterior position (FIG. 7B), and can be positioned in one more locations between the illustrated anterior and posterior positions. FIG. 7C shows a side view of the dental appliance from inside the dental arcade, showing the lingual right side of the attachment mechanism and the constraining mechanism.

In one or more alternative embodiments of the dental appliance having a forward-oriented constraining mechanism, the dorsal section of the component can be curved and not feature a central buckle. An example implementation of the forward oriented constraining mechanism without a buckle is shown at FIGS. 8A-C. If need be, a minor lateral adjustment can be made by somewhat flattening the curve of the dorsal portion of the component.

FIG. 8A shows the length, in mm, of exemplary forward-oriented constraining mechanisms without a buckle. In particular, FIG. 8A shows the length of a forward-oriented short constraining mechanism (FwS) and a forward-oriented long constraining mechanism (FwL) in accordance with one or more embodiments.

FIGS. 8B-C show a perspective view (8B) and a bottom view (8C) of the exemplary forward-oriented constraining mechanisms without a buckle, in accordance with at least one embodiment. As shown in FIGS. 8B-C, the constraining mechanism comprises substantially the same features as the embodiment of FIGS. 6A-C (and therefore, like elements are numbered alike), including a C-shaped component 305 having a dorsal section 310 with an apex 325. In this embodiment, the center portion of the dorsal section 310 is covered by a coating resin 315. In one or more implementations, the resin coating 315 is approximately 1 mm thick around the wire. The constraining mechanism 300 also comprises a pair of wired ends 335 configured to removably connect the constraining mechanism 300 with the attachment mechanism via a fastening mechanism. More specifically, as with the prior embodiment, the pair of wired ends 335 are bent and thus configured to insert into the fastening mechanism from a rearward direction, which allows for increased flexibility and adjustability. In one or more embodiments, the component is angled downward relative to base of the attachment mechanism in the range of 0° to 30°, and in certain implementations, can be preset at a downward angle of e.g., 15°.

With continued reference to FIGS. 8B-C, in this embodiment, the forward-oriented constraining mechanism 300 also comprises a pair of horizontal loops 340, one on each side of the component 305. The horizontal loops 340 are part of the component (e.g., made of wire) and are not coated in resin. The horizontal loops 340 allow for transverse (widening) adjustment of the constraining mechanism in an effort to not hinder the lateral expansion of the dental arcade palate elaborated by the lateral edges of the constrained tongue. Specifically, when the palate of the user is too narrow, the pressure exerted by the tongue under constraint from the component 305 can cause the dental arcade to enlarge laterally over time as the tongue is flattened. More specifically, when wearing the device, the dental arcade and consequently the palate can widen as the tongue flattens because pressure on the tongue from the component can cause the tongue to exert a lateral force on the lingual side of the molar teeth. Accordingly, the component 305 (e.g., wire) must be flexible enough such that the expansion of the dental arcade by the tongue is not hindered. When there is a central buckle, the component can be laterally expanded by opening the central buckle. If, as in this embodiment, there is no central buckle, horizontal loops 340 can be used to enlarge the component manually to the same effect. Either loops or buckle allow for lateral enlargement of the dental arcade without the component directly exerting either a restraining or an expanding lateral force on the molar teeth. In one or more implementations, the constraining mechanism 300 having a component 305 with a forward orientation can be adjusted laterally by up to approximately 5 mm, by slightly opening the central buckle if one is provided . This can be done by an orthodontist or other dental professional. Optionally, when present, as in FIG. 7A, the buckle can be encased (e.g., by dipping) in a "bead" or coating of resinous material for added comfort (not shown). A bead or coating similar to 315 in Fig. 8A-C but sufficient to coat the buckle can be cut for adjustment and a new coating can be put on.

In at least one alternative embodiment, the constraining mechanism can also feature a pair of vertical (or oblique) loops (stops) (not shown), one on each side of the component 305. In FIG 8C, the vertical loops would be located between horizontal loops 340 and the bend in the component where ends 335 begin. The pair of oblique or vertical loops are also part of the component and allow for additional vertical flexibility in that the component 305 can be adjusted up or down up to 30° from the occlusal plane. In one or more implementations, the vertical loops can include a thin (e.g., 1mm) silicone coating around the loops. It should be noted that the embodiments that feature the central buckle do not have other horizontal loops in the constraining mechanism.

### Fastening Mechanism

In one or more embodiments, and as shown in FIG. 7, the constraining mechanism 300 is removably secured to the attachment mechanism 200 via a fastening mechanism 400. The fastening mechanism 400 can include a tightening means such as a screw or nut-and-bolt type combination mechanism. Exemplary components of the fastening mechanism 400 and tightening means are shown at FIGS. 9A-C, in accordance with one or more embodiments.

As shown in FIG. 9A, in at least one embodiment, the fastening mechanism can comprise a tightening (tensioning) means, which includes a pair of miniature tube nuts 405 (these nuts are commercially available) each featuring an aperture 410 and a bolt 415 for connecting the respective ends of the constraining mechanism wire to the attachment mechanism. The fastening mechanism is secured to the second surface of the attachment mechanism by means of a metal foot, 422, which is embedded into the material of the attachment mechanism. More specifically, the apertures 410 receive the respective ends (335, as shown, e.g., in FIG. 7A or 535 as shown, e.g., in FIG. 12A or 12B) of the constraining mechanism and bolts 415 help to hold the respective ends 335 in place as by tightening the bolt to capture (e.g., pinch) the wire end. In one or more embodiments of the forward-oriented constraining mechanism, the pair of wire ends are bent back towards the longitudinal direction of the constraining mechanism and thus are configured to insert into the fastening mechanism 400 from a rearward direction even though most of the constraining mechanism is located in a position anterior to the fastening mechanism *(see* FIG. 7A). This bent configuration of the wired ends of constraining mechanism allows for increased flexibility and adjustability with regards to the fastening mechanism. As such, the bent wired ends 335 can slide back and forth within the tube nut 405 as part of the adjustment mechanism for adjusting the position of the component relative to the rear molars. Vertical adjustment of the component can be achieved as described above by using vertical loops or by altering the angle of ends 335 to C-shaped portion of the component, thereby adjusting the limitation in the movement of the tongue. This arrangement works with both forward and rearward oriented constraining mechanisms.

In an exemplary embodiment, the ends of the constraining mechanism are inserted into the fastening mechanism so that the component is at a preset downward angle of e.g.,15° relative to the plane defined by the ends of the component. In one or more implementations, the apertures 410 are sized and shaped to receive a wire with a diameter of approximately 0.5 to approximately 1.2 mm, preferably within the range of about 0.7 to 0.9 mm. However, in one or more implementations, the apertures can be sized and shaped to receive wires of other sizes.

In one or more embodiments, the tube nuts 405 can be welded, embedded in, or otherwise attached to the lingual portion of the respective second surfaces of the circumferential band of the attachment mechanism. More specifically, in at least one embodiment, the tube nuts 405 can be attached, in a vertical orientation, on the second surfaces of the circumferential band adjacent to the mesial side of the respective second molars *(see* FIG. 7C). In other implementations, the tube nuts can be attached on the second surfaces of the circumferential band adjacent to the mesial side of the first or third molars. FIGS. 7A-B show the tube nut 405 positioned approximately adjacent to the middle of the first molar, however, in other embodiments, the tube nut 405 can positioned at other positions along the lingual side of the molars, such as the position adjacent to the mesial end of the second molar *(see* FIG. 7C). There is thus a range of positions along the left or right second surface wherein the tube nut 405 may be secured to the second surface.

In at least one embodiment, the fastening mechanism can comprise a foot 422 (protrusion) which is embedded in the resin of one of the first and second surfaces of the attachment mechanism to secure the fastening mechanism on to the attachment mechanism. In one or more embodiments, the fastening mechanism can further include a metal strip or foot 422 welded onto the tube nuts 405 and embedded in the resinous material of the lingual part of the attachment mechanism. Foot 422 is thus a protrusion that extends radially outward from the tube nut body to provide a structure that can be attached to the resinous structure. In at least one embodiment, the tightening means comprises a clamp.

With reference to FIG. 9B, the fastening mechanism can comprise a pair of sheaths 420, the two sheaths attached to the lingual portion of respective second surfaces of the circumferential band by means of a metal foot 422. FIG. 9B illustrates one sheet 420. The sheaths 420 each comprise a groove 425 that is sized and shaped to receive and hold the respective ends 335 or 535 of the constraining mechanism wire. Thus, the grooves 425 allow for back and forth sliding of the ends of the constraining mechanism wire within the confines of the groove 425. Accordingly, in one or more embodiments, the sheaths 420 (and their grooves 425) can be approximately 3mm long to receive approximately 3mm of the ends of the constraining mechanism. However, in other implementations, the sheaths can be shortened or lengthened to accommodate a constraining mechanism end of another length.

With reference to the embodiment of FIG. 9B, the tightening means is configured to further secure the end of the constraining mechanism (e.g., 335 or 535) that is inserted into the groove 425. The tightening means serves as part of the fastening mechanism for the dental appliance, as it is used to secure the ends of the constraining mechanism inserted into the groove 425.

In one or implementations, the sheath 420 can also act as part of the tightening means. Specifically, in an embodiment in which the component of the constraining mechanism has a vertical loop on each end portion (as discussed in greater detail below) a rubber band can be used to wrap around one end of the sheath and the other end can be tensioned and looped around its respective constraining mechanism vertical or oblique loop to affix and tighten the constraining mechanism to the sheath. FIG. 9C shows an exemplary rubber band 430 wrapped around a loop of the constraining mechanism.

In one or more embodiments, a sheath 420 is permanently affixed (e.g., embedded, directly, or via foot 422 or other structure) to the resinous material of a lingual portion of a second side of the attachment mechanism. The wire ends of the constraining mechanism sheath fit tightly into the attachment means sheaths, holding the constraining mechanism in place. The constraining mechanism can be removed by removing the rubber band pulling the wire ends out of the groove of the sheaths.

### Rearward-Oriented Constraining Mechanism

An exemplary constraining mechanism having a rearward (backward) orientation is shown at FIGS. 10A-C. FIG. 10A shows the length, in mm, of exemplary backward-oriented constraining mechanisms. In particular, FIG. 10A shows the length of a backward-oriented short constraining mechanism (BwS) and a backward-oriented long constraining mechanism (BwL) in accordance with one or more embodiments.

FIGS. 10B-C show a back view (B) and an elevated front view (C) of the exemplary backward-oriented constraining mechanism. As shown in FIGS. 10B-C, in this embodiment, the constraining mechanism 500 has a component 505 with a rearward orientation. The component 505 is generally C-shaped and comprises a dorsal section 510. Further, in this embodiment, the component 505 is generally a wire in which a center portion of the component [i.e., most of the dorsal section 510]) can be covered by a coating resin 515. In one or more embodiments, the resin coating 515 is approximately 1-2 mm thick around the wire. In certain implementations, the coating 515 can be slightly thicker near the apex 525 of the dorsal section 510 relative to the other coated sections. In one or more implementations, the constraining mechanism 500 can be sized and shaped to generally follow the shape of the back of the user's dental arcade. Further, the coating resin 515 can be shaped in such a way that it prevents any sharp edges so as not to be painful to the tongue of the user. In one or more embodiments, the component is angled downward relative to base of the attachment mechanism in the range of 0 to 30°, and in certain implementations, can be preset at a downward angle of 15°. Instead of the metal wire and resin combination, the component can be made of a polymeric wire or tube material having the requisite strength and flexibility (such as a polyether ether ketone (PEEK) polymer).

The rearward-oriented constraining mechanism 500 also comprises a pair of wired ends 535 configured to removably connect the constraining mechanism 500 with the attachment mechanism via a fastening mechanism. In this embodiment, the pair of wire ends 535 are configured to insert into the fastening mechanism from a rearward direction, allowing for increased flexibility and adjustability. In one or more embodiments, the shape and positioning of the rearward-oriented constraining mechanism 500 within the mouth can be adjusted manually by a dental health professional. For example, the rearward-oriented constraining mechanism 500 can be adjusted so as to be placed as far back as possible on tongue (e.g., close to the lingual V zone), thereby increasing the effect of the dental appliance at or near the pharyngeal zone (for correcting certain conditions), but avoid nausea or the gag reflex. The downward angle can be adjusted by altering the angle of the wire ends 535 to the C-shaped section, component 505. The angle can be measured by reference to the occlusal plane or to the plane defined by wire ends 535.

Another embodiment of the constraining mechanism having a rearward (backward) orientation is shown at FIGS. 11A-C, which the constraining mechanism has vertical or oblique loops. FIG. 11A shows the length, in mm, of exemplary backward-oriented constraining mechanisms with vertical loops, in accordance with at least one embodiment. In particular, FIG. 11A shows the length of an exemplary backward-oriented short constraining mechanism (BwS) with vertical or oblique loops and a backward-oriented long constraining mechanism (BwL) with vertical or oblique loops in accordance with one or more embodiments.

FIGS. 11B-C show a back view (B) and an elevated front view (C) of the exemplary backward-oriented constraining mechanism with vertical or oblique loops. As shown in FIGS. 11B-C, in this embodiment, the constraining mechanism 500 has substantially the same features and attributes as those shown in the embodiment of FIGS. 10A-C, but also includes a pair of vertical or oblique loops 540, one on each side of the component 505. In the illustrated embodiment, the vertical or oblique loops 540 are part of the component 505 and are not coated in resin and allow for widening of the constraining mechanism in an effort to alter the angle of component 505 to the occlusal plane of the person or to the basal plane of the constraining mechanism defined by the wire ends 535. This adjustment can be effected by varying the vertical loops to bend the component downwards by reference to the occlusal plane to an angle from zero up to 30°. The adjustment can be made in embodiments without loops by bending the wire ends of the constraining mechanism to create or adjust this angle. Alternatively, another constraining mechanism having a preset angle determined to be appropriate can be substituted.

FIGS. 12A-D show an exemplary dental appliance featuring the rearward-oriented constraining mechanism 500, in accordance with one or more embodiments of the present application. In particular, FIGS. 12A-C show the rearward-oriented constraining mechanism 500 attached to the attachment mechanism 200 via the fastening mechanism 400. In this embodiment, the attachment mechanism features wings 235, where the right wing features a sensor 230. FIG. 12A shows a shorter embodiment of the constraining mechanism 500 (BwS), where the apex 525 of the constraining mechanism 500 is located adjacent to the distal end of the second molars. FIG. 12B shows a longer embodiment of the constraining mechanism 500 (BwL), where the apex 525 of the constraining mechanism 500 is located behind (past the distal end of) the third molars. FIG. 12C shows a side view of the longer embodiment of the constraining mechanism 500 and shows the lingual right side of the wing 235 and the sensor 230. In at least one implementation, as shown in FIG. 12B, C, the embedded sheath 420 of the fastening mechanism 400 in the lingual portion of the second surface can extend substantially through the entire length of the second surface, which allows for greater longitudinal adjustment of the constraining mechanism. FIG. 12D is directed to embodiments with a rearward oriented component as in FIG. 12A wherein the component has a central buckle 520 with an opening 530 for lateral expansion of the constraining mechanism as was described in connection with FIG. 7B for the forward oriented component.

In one or more implementations, the dental appliance of the present application can have one or more additional features for customizing the dental appliance for a particular user or for correcting a particular type of condition. For instance, in one or more embodiments, the dental appliance can include covers on the vestibular side of all the teeth in the upper dental arcade, and covers on lingual side of the all the upper molars. These covers do not cover any biting surface of the upper teeth and thus does not hinder full occlusal contact of all the teeth. In one or more implementations, the attachment of the dental appliance to the dental arcade can be accomplished using minimal clamping forces only on the upper incisors and the last upper molars. This minimal clamping force is enough to hold the dental appliance in place, and thus no other transversal, sagittal, or vertical force is required. As such, in this embodiment, the dental appliance can be used with natural fragile or implanted dentition or dentures.

In one or more implementations, the resin coatings of the attachment mechanism and/or constraining mechanism can be treated with antimicrobial surface agents, such as, antibacterial surface agents or antifungal surface agents.

As used herein, the term "adjustment mechanism" refers to portions of the constraining mechanism and/or attachment mechanism that are used for adjusting the angle of the component of the constraining mechanism relative to the occlusal plane of the person when the appliance is worn. These adjustments to the component control the extent of limitation of the movement of the tongue. The term "adjustment mechanism" also includes portions of the constraining mechanism and/or attachment mechanism that are used for adjusting the position of the component along a longitudinal axis of the appliance. For instance, the constraining mechanism can be slidably mounted onto the adjustment mechanism (e.g., the ends of constraining mechanism are slidably mounted to the fastening mechanism including the sheath held within the second surface of the attachment mechanism) to permit adjustment of the position of the component along a longitudinal axis of the appliance *(see* FIGS. 7A-B). Accordingly, the adjustment mechanism can comprise the fastening mechanism (including the tightening means), the wire ends of constraining mechanism, and the second surface of the attachment mechanism, which in certain embodiments, can be configured to hold portions of the fastening mechanism (e.g., the sheath). In various embodiments, the adjustment mechanism can further include the pair of horizontal wire loops or central buckle for optional lateral expansion of the component *(see* FIG. 8), a pair oblique or vertical loops located at an end section of the constraining mechanism for adjusting the position of the apex of the component upwards or downwards, or the adjustment mechanism can comprise no loops and the vertical adjustment of the apex of the component can occur by altering the angle of the end wires of the constraining mechanism or by substituting another component with a different preset angle.

### Treatment

FIGS. 13A-D show the positioning of the component of the constraining mechanism to impose a mild constraint on the tongue in accordance with one or more embodiments. More specifically, FIG. 13A shows a mild constraint position on the tongue by reference to the dental arcade and vertical distance from the occlusal plane for a long, forward-oriented constraining mechanism (FwL) and FIG. 13B shows a mild constraint position on the dental arcade for a short, forward-oriented constraining mechanism (FwS). Similarly, FIG. 13C shows a mild constraint position on the tongue by reference to the dental arcade and vertical distance from the occlusal plane for a short, rearward-oriented constraining mechanism (BwS) and FIG. 13D shows a mild constraint position on the tongue by reference to the dental arcade for a long, rearward-oriented constraining mechanism (BwL). The notation "mm" signifies the vertical distance of the apex of the component from the occlusal plane; the notation "Di4" means distal end of tooth 4 and signifies how far forward the component apex reaches; similarly, "Me8" means mesial end of tooth 8. The tooth numbering system is the European system wherein numbering starts from the middle incisor (1) and ends with the last molar (8) on the same side of the upper dental arcade. Thus, tooth 4 is a first premolar. The same notations apply to Figs 14A-D (depicting strong constraint on the tongue), 15A, 16A, 17A and 18A. Together, the foregoing distance and dental arcade reference for the apex provide a measure of the severity (intensity) of the constraint on the tongue as well as pinpoint where on the tongue the constraint is focused.

FIGS. 14A-D show the positioning of the component of the constraining mechanism to impose a strong constraint on the tongue in accordance with one or more embodiments. In particular, FIG. 14A shows a strong constraint position on the tongue by reference to the dental arcade and vertical distance from the occlusal plane for a long, forward-oriented constraining mechanism (FwL) and FIG. 14B shows a strong constraint position on the tongue by reference to the dental arcade and vertical distance from the occlusal plane for a short, forward-oriented constraining mechanism (FwS). Similarly, FIG. 14C shows a strong constraint position on the tongue by reference to the dental arcade and vertical distance from the occlusal plane for a short, rearward-oriented constraining mechanism (BwS) and FIG. 14D shows the maximum constraint position on the tongue by reference to the dental arcade for a long, rearward-oriented constraining mechanism (BwL).

FIGS. 15A-F show an exemplary treatment plan for treating a tongue condition associated with snoring obstructive sleep apnea (OSA), in accordance with one or more embodiments. FIG. 15A shows an exaggerated schematic of the shape of an abnormally positioned tongue representing a tongue dysfunction associated with obstructive sleep apnea. In this condition, as shown in FIG. 15A, the patient has an abnormally positioned tongue with a high and posterior posture and has a palate with a large deep cavity which may put pressure on the nasal fossae. This aberrant tongue posture and associated palatal malformation believed to be caused by faulty (so-called atypical) swallowing movements of the tongue, result in an abnormally high tongue posture at rest, and are associated with OSA. FIG. 15B shows a diagram of the locations of the apex of the component of the constraining mechanism for each of the four steps (represented by the series of dots as well as numbers corresponding to each particular step) for treating the tongue condition shown in FIG. 15A. As shown in FIG. 15B, the apex of the component of the constraining mechanism moves progressively backward and downward with each treatment step in an effort to flatten the tongue, place transverse force on the molars from the inside, correct the tongue's swallowing movement as well as the tongue's resting posture, which may permit remodeling of the palate to a shallower, closer to normal morphology. The length of time that each treatment step is implemented can vary from patient to patient, depending on the particular oral morphology of the patient as well as other factors discussed *infra.*

FIG. 15C shows the positioning of the apex of the component of the constraining mechanism relative to the tongue for treatment step one. As shown, the component used for step one is a forward-oriented short ("FwS") component, and the apex of the component is at a location such that an anterior portion of the middle of the tongue is constrained in a downward fashion.

FIG. 15D shows the positioning of the apex of the component of the constraining mechanism for treatment step two. As shown, the component used for step two is a backward-oriented short ("BwS") component, and the apex of the component is at a location such that a posterior portion of the middle of the tongue is constrained in a downward fashion.

FIG. 15E shows the positioning of the apex of the component of the constraining mechanism for treatment step three. As shown, the component used for step three is a backward-oriented long ("BwL") component, and the apex of the component is located adjacent to the distal portion of the last molar such that a portion of the back of the tongue is constrained in a downward fashion.

FIG. 15F shows the positioning of the apex of the component of the constraining mechanism for treatment step four. Like step three, the component used for step four is a BwL component, and the apex of the component is located at a position distal to the last molar such that a portion of the back of the tongue is constrained in a downward fashion.

FIGS. 16A-G show an exemplary treatment plan for treating a tongue condition associated with malocclusion and/or snoring, in accordance with one or more embodiments. FIG. 16A shows an exaggerated schematic of the shape of an abnormally positioned tongue associated with malocclusion and/or snoring. In this condition, as shown in FIG. 16A, the patient has an abnormally positioned tongue with a high anterior posture and has a deep, narrow palate. The tongue's aberrant position and in some cases morphology is thought to be associated with dysfunctions such as atypical swallowing movements and with abnormally tongue posture at rest. These dysfunctions are believed to contribute to dental malocclusion, Obstructive Sleep Apnea (OSA) or snoring during sleep. FIG. 16B shows a diagram of the locations of the apex of the component of the constraining mechanism for each of the five steps (represented by the series of dots as well as numbers corresponding to each particular step) for treating the tongue condition shown in FIG. 16A. As shown in FIG. 16B, the apex of the component of the constraining mechanism is moved progressively backward and downward with each treatment step in an effort to flatten the tongue and correct the tongue's swallowing movement and resting posture. Again, the length of time that each treatment step is implemented can vary from patient to patient, depending on the particular morphology of the patient and other factors discussed infra.

FIG. 16C shows the positioning of the apex of the component of the constraining mechanism relative to the tongue for treatment step one. As shown, the component used for step one is a forward-oriented long ("FwL") component, and the apex of the component is at a location such that a portion the tongue just posterior to the tip is constrained in a downward fashion.

FIG. 16D shows the positioning of the apex of the component of the constraining mechanism for treatment step two. As shown, the component used for step two is an FwS component, and the apex of the component is at a location such that a portion of the middle of the tongue (approximately adjacent to the mesial end of the first molar) is constrained in a downward fashion.

FIG. 16E shows the positioning of the apex of the component of the constraining mechanism for treatment step three. As shown, the component used for step three is a BwS component, and the apex of the component is located adjacent to the distal end of the second molar such that a back portion of the middle of the tongue is further constrained in a downward fashion.

FIG. 16F shows the positioning of the apex of the component of the constraining mechanism for treatment step four. As shown, the component used for step four is a BwL component, and the apex of the component is located at a position adjacent to the distal end of the last molar such that a back portion of the tongue is still further constrained in a downward fashion.

FIG. 16G shows the positioning of the apex of the component of the constraining mechanism for treatment step five. Like step four, the component used for step five is a BwL component, and the apex of the component is located at a position distal to the last molar such that a portion of the back of the tongue is constrained in a downward fashion.

FIGS. 17A-D show an exemplary treatment plan for treating a tongue condition associated with malocclusion and/or snoring, in accordance with one or more embodiments. FIG. 17A shows an exaggerated schematic of the shape of an abnormally positioned tongue associated with malocclusion or snoring or both. In this condition, as shown in FIG. 17A, the patient has an abnormally positioned tongue with a high anterior posture and has a deep, narrow palate. This altered morphology is associated with dysfunctions such as atypical swallowing movement and/or tongue interposition between dental arches. FIG. 17B shows a diagram of the locations of the apex of the component of the constraining mechanism for the two steps for treating the tongue condition shown in FIG. 17A. Again, the length of time that each treatment step is implemented can vary from patient to patient, depending on the oral morphology of the patient and other factors discussed infra.

FIG. 17C shows the positioning of the apex of the component of the constraining mechanism relative to the tongue for treatment step one. As shown, the component used for step one is an FwL component, and the apex of the component is at a location such that a portion the tongue just posterior to the tip is constrained in a downward fashion.

FIG. 17D shows the positioning of the apex of the component of the constraining mechanism for treatment step two. As shown, the component used for step two is an FwS component, and the apex of the component is at a location such that a portion of the middle of the tongue (approximately adjacent to the first molar) is constrained in a downward fashion.

Finally, FIGS. 18A-D show an exemplary treatment plan for treating a tongue condition associated with persistent atypical swallowing, in accordance with one or more embodiments. FIG. 18A shows an exaggerated schematic of the shape of an abnormally positioned tongue associated with this dysfunction. In this condition, as shown in FIG. 18A, the patient has an abnormally positioned tongue with a high anterior posture, but a normal palate. This altered tongue morphology is believed to be caused by persistent atypical swallowing wherein the tongue is in an incorrect position during swallowing. FIG. 18B shows a diagram of the locations of the apex of the component of the constraining mechanism for the two steps for treating the tongue condition shown in FIG. 18A. Like the previous exemplary treatment regimens, the length of time that each treatment step is implemented can vary from patient to patient, depending on the patient's oral morphology and other factors discussed infra.

FIG. 18C shows the positioning of the apex of the component of the constraining mechanism relative to the tongue for treatment step one. As shown, the component used for step one is an FwS component, and the apex of the component is at a location just anterior to the first molar, such that a middle portion the tongue is constrained in a downward fashion.

FIG. 18D shows the positioning of the apex of the component of the constraining mechanism for treatment step two. As shown, the component used for step two is also an FwS component, and the apex of the component is at a location adjacent to the first molar such that a portion of the middle of the tongue (approximately adjacent to the first and second molars) is constrained in a downward fashion.

### Duration of Treatment

The treatment duration and regimen depends significantly upon the patient's oral morphology and the severity of the dysfunction. Other factors, such as the patient's age, overall physical condition, intrinsic responsiveness to the treatment and tolerance of the treatment also play a role. Generally, the treatment begins with the patient wearing the appliance several hours at a time during the day and during sleep for 3 to 4 weeks, then switching to wearing it during sleep only. Duration of treatment is typically from between about 3 to about 10 months. If both a forward and a backward component need to be used, they are used seriatim for at least about 3 weeks each until the last step is reached which will last for the remaining time of the treatment. Adjustments to the confinement imposed upon the tongue (angle and sagittal length of the apex of the component, the latter implemented by switching to a longer or shorter component and by varying the vertical angle of same by reference to the occlusal plane, as needed) and/or to the basal plane of the component defined by the wire ends can be made periodically. If necessary, treatment can be repeated after a period of pause.

### Dental Applicant Kit

As can be appreciated by the foregoing examples, a program for limiting movement of the tongue can consist of, at least in one exemplary embodiment, use of a plurality of different dental appliances that are used in succession according to a patient specific plan. Accordingly, the plurality of devices can be supplied as part of a customized kit or assembly for use by the patient as part of a customized program or plan for limiting movement of the tongue. It will equally be understood that in other customized programs, only a single dental appliance may be needed to obtain the desired results for this specific patient and therefore, a kit of plural dental appliances is not needed.

## Claims

1. A dental appliance for limiting movement of a central zone or posterior zone of the tongue of a person while allowing an anterior zone and lateral edges of the tongue to perform movements necessary for speech and swallowing, the dental appliance comprising:
an attachment mechanism (200) to attach the dental appliance onto teeth within the mouth of the person, wherein the attachment mechanism comprises
a substantially but not fully circumferential band (205) having:
a first surface (210) sitting on a vestibular side of a dental arcade of the person;
a pair of left and right second surfaces (215), each second surface in opposition to the first surface and adapted to be seated along at least one rear corresponding, left or right, tooth on the lingual side of said at least one tooth, said second surfaces adapted to extend along the lingual side of a limited number of rear teeth of the person,
the attachment mechanism is adapted to snap onto the dental arcade of the person to releasably secure the appliance on the dental arcade;
a connector (220) joining the first surface and the second surface; and
a constraining mechanism (300; 500) secured to the attachment mechanism and having a component (305; 505) that spans between the pair of second surfaces, the component having a forward or rearward orientation and being configured to seat at approximately an occlusal plane of the person or at an angle thereto to limit movement of the central zone of the tongue when in the forward orientation or the posterior zone of the tongue when in the backward orientation and thereby to confine the range of motion of the tongue drawing it forward and/or downward, while allowing at least the lateral edges of the tongue and, correspondingly, either its posterior zone and tip or its anterior zone to perform movements necessary for speech and swallowing;
wherein when the component has a forward orientation the constraining mechanism is centrally positioned relative to the second surfaces and a forward portion of the band so as to seat at a central portion of the tongue;
**characterised in that** the constraining mechanism is removably secured to the attachment mechanism, and **in that** the constraining mechanism or the attachment mechanism comprises an adjustment mechanism for adjusting: (i) the angle of the component relative to the occlusal plane of the person when the appliance is worn and thereby controlling the extent of limitation of the movement of the tongue; or (ii) the position of the component along a longitudinal axis of the appliance and thereby controlling the zone of the tongue wherein the constrain is to be applied; or (iii) both said angle and said position;
wherein the constraining mechanism is secured to the attachment mechanism by a fastening mechanism (400), wherein the fastening mechanism comprises a nut and screw or a nut and bolt combination, wherein each end of the constraining mechanism is slidably mounted and secured to the fastening mechanism in an adjustable manner.

2. The dental appliance of claim 1, wherein the component spans between the left and right second surfaces at a rear portion of the band proximate the pair of second surfaces, optionally wherein the constraining mechanism has one end connected to the left second surface and another end connected to the right second surface.

3. The dental appliance of claim 1, wherein the substantially circumferential band comprises resinous material that is in contact with the dental arcade when the appliance is worn, optionally, wherein the first surface of the attachment mechanism conforms to and is in registry with the gum line of the vestibular surface of the person's upper dental arcade, further optionally, wherein the first surface of the attachment mechanism covers at least a majority of the vestibular surface of the dental arcade from the gum line down but essentially does not cover the occlusal surface of teeth in said arcade.

4. The dental appliance of claim 1, wherein the substantially circumferential band further comprises a wing (235) attached to a lingual side of the second surface, the wing bearing a sensing device, optionally embedded in the wing, optionally, wherein the sensing device is a temperature or pH or sound sensor, optionally, further comprising at least one of the following:
the wing comprises a resinous material; and
the sensing device is embedded in the resinous material and disposed so as not to touch a palate or teeth of the person when the appliance is worn by the person.

5. The dental appliance of claim 1, wherein the component is generally C-shaped with a dorsal section of the component disposed transversely to a longitudinal axis of the appliance and responsible for limiting the movement of the tongue, and wherein the constraining mechanism has a first and second end for securing the constraining mechanism to the attachment mechanism of the corresponding second surface via the fastening mechanism.

6. The dental appliance of claim 1, wherein the constraining mechanism comprises a wire optionally comprising a center section enveloped in a polymeric material.

7. The dental appliance of claim 1, further comprising an arcuate support member (225) for increased rigidity or strength of the appliance, wherein the member spans the second surfaces and is disposed upwards and following a contour of the palate without touching the teeth or palate of the person when the appliance is worn.

8. The dental appliance of claim 1, wherein the component comprises a wire with a central buckle for flexibility and optional lateral expansion of the component, optionally, wherein the fastening mechanism is embedded in one of the second surfaces of the attachment mechanism to secure the fastening mechanism to the attachment mechanism.

9. The dental appliance of claim 1, wherein the fastening mechanism permits the position of the component to be longitudinally adjusted forward or rearward.

10. The dental appliance of claim 6, wherein the constraining mechanism is slidably mounted onto the adjustment mechanism via the fastening mechanism to permit adjustment of the position of the component along a longitudinal axis of the appliance.

11. The dental appliance of claim 1, wherein the component of the constraining mechanism is angled by reference to the occlusal plane of the person when the appliance is worn or a plane defined by the ends of the constraining mechanism, with the angle being within the range from approximately 0 to approximately 30° or +15° to approximately -15° when the appliance is worn by the person.

12. The dental appliance of claim 1, wherein the component further comprises a wire with a central buckle for flexibility and optional lateral expansion of the component.

13. The dental appliance of claim 1, wherein the fastening mechanism comprises at least one foot (422) which is affixed or embedded in the corresponding second surface of the attachment mechanism to secure the fastening mechanism on to the attachment mechanism, or, wherein the fastening mechanism permits the position of the component to be longitudinally adjusted forward and rearward.

## Patentansprüche

1. Dentalvorrichtung zur Einschränkung der Bewegung eines mittleren Bereichs oder hinteren Bereichs der Zunge einer Person, wobei ein vorderer Zungenbereich und seitliche Ränder der Zunge weiterhin Bewegungen durchführen können, die für das Sprechen und Schlucken notwendig sind, wobei die Dentalvorrichtung Folgendes umfasst:
einen Befestigungsmechanismus (200) zum Befestigen der Dentalvorrichtung an Zähnen im Mund der Person, wobei der Befestigungsmechanismus ein im Wesentlichen, aber nicht vollständig, umlaufendes Band (205) umfasst, das Folgendes aufweist:
eine erste Fläche (210), die auf der vestibulären Seite eines Zahnbogens der Person liegt;
ein Paar linker und rechter zweiter Flächen (215), wobei jede zweite Fläche der ersten Fläche gegenüberliegt und so angepasst ist, dass sie entlang mindestens eines entsprechenden, linken oder rechten, hinteren Zahns auf der lingualen Seite dieses mindestens einen Zahns aufsitzt, wobei die zweiten Flächen so angepasst sind, dass sie entlang der lingualen Seite einer begrenzten Anzahl von hinteren Zähnen der Person verlaufen,
wobei der Befestigungsmechanismus so angepasst ist, dass er am Zahnbogen des Person einrastet, um die Vorrichtung lösbar am Zahnbogen anzubringen;
ein Verbindungsstück (220), das die erste Fläche und die zweite Fläche miteinander verbindet; und
einen am Befestigungsmechanismus angebrachten Beschränkungsmechanismus (300; 500), der eine Komponente (305; 505) aufweist, die sich zwischen den beiden zweiten Flächen erstreckt, wobei die Komponente eine Ausrichtung nach vorne oder nach hinten aufweist und so konfiguriert ist, dass es in etwa auf der Okklusionsebene der Person oder in einem Winkel dazu aufsitzt, um die Bewegung des mittleren Bereichs der Zunge bei der Vorwärtsausrichtung oder des hinteren Bereichs der Zunge bei der Rückwärtsausrichtung einzuschränken, und dadurch den Bewegungsspielraum der Zunge, der sie nach vorne und/oder nach unten zieht, zu begrenzen, wobei zumindest die seitlichen Ränder der Zunge und entsprechend entweder ihr hinterer Bereich und ihre Spitze oder ihr vorderer Bereich Bewegungen durchführen können, die für das Sprechen und Schlucken notwendig sind;
wobei bei einer Vorwärtsausrichtung der Komponente der Beschränkungsmechanismus mittig zu den zweiten Flächen und einem vorderen Abschnitt des Bandes positioniert ist, um an einem mittleren Abschnitt der Zunge aufzusitzen;
**dadurch gekennzeichnet, dass** der Beschränkungsmechanismus abnehmbar am Befestigungsmechanismus angebracht ist und dass der Beschränkungsmechanismus oder der Befestigungsmechanismus einen Einstellmechanismus umfasst, um Folgendes einzustellen: (i) den Winkel der Komponente relativ zur Okklusionsebene der Person beim Tragen der Vorrichtung und dadurch das Ausmaß der Einschränkung der Bewegung der Zunge zu steuern; oder (ii) die Position der Komponente entlang einer Längsachse der Vorrichtung, und dadurch den Bereich der Zunge zu steuern, in dem die Beschränkung angewendet werden soll; oder (iii) sowohl den Winkel als auch die Position;
wobei der Beschränkungsmechanismus mittels eines Fixiermechanismus (400) am Befestigungsmechanismus angebracht ist, wobei der Fixiermechanismus eine Mutter und Schraube oder eine Kombination aus Mutter und Schraube umfasst und wobei jedes Ende des Beschränkungsmechanismus verschiebbar montiert und auf einstellbare Weise am Fixiermechanismus angebracht ist.

2. Dentalvorrichtung nach Anspruch 1, wobei sich die Komponente zwischen der linken und rechten zweiten Fläche an einem hinteren Abschnitt des Bandes in der Nähe der beiden zweiten Flächen erstreckt, wobei der Beschränkungsmechanismus optional ein Ende aufweist, das mit der linken zweiten Fläche verbunden ist, und ein anderes Ende, das mit der rechten zweiten Fläche verbunden ist.

3. Dentalvorrichtung nach Anspruch 1, wobei das im Wesentlichen umlaufende Band Harzmaterial umfasst, das beim Tragen der Vorrichtung den Zahnbogen berührt, wobei optional die erste Fläche des Befestigungsmechanismus der Zahnfleischlinie der vestibulären Fläche des oberen Zahnbogens der Person entspricht und mit dieser fluchtet, wobei optional weiter die erste Fläche des Befestigungsmechanismus mindestens einen Großteil der vestibulären Fläche des Zahnbogens von der Zahnfleischlinie abwärts bedeckt, die Okklusionsfläche der Zähne in diesem Zahnbogen jedoch im Wesentlichen nicht bedeckt.

4. Dentalvorrichtung nach Anspruch 1, wobei das im Wesentlichen umlaufende Band weiter einen Flügel (235) umfasst, der an einer lingualen Seite der zweiten Fläche befestigt ist, wobei der Flügel eine Sensorvorrichtung trägt, die optional in den Flügel eingebettet ist, wobei es sich bei der Sensorvorrichtung optional um einen Temperatur-, pH- oder Schallsensor handelt, der optional weiter mindestens eines der folgenden Elemente umfasst:
der Flügel umfasst ein harzartiges Material; und
die Sensorvorrichtung ist in das Harzmaterial eingebettet und so angeordnet, dass sie beim Tragen der Vorrichtung durch eine Person weder den Gaumen noch die Zähne der Person berührt.

5. Dentalvorrichtung nach Anspruch 1, wobei die Komponente im Allgemeinen C-förmig ist, wobei ein dorsaler Teilabschnitt der Komponente quer zur Längsachse der Vorrichtung angeordnet ist und für die Einschränkung der Zungenbewegung zuständig ist, und wobei der Beschränkungsmechanismus ein erstes und ein zweites Ende aufweist, um den Beschränkungsmechanismus über den Fixiermechanismus am Befestigungsmechanismus der entsprechenden zweiten Fläche anzubringen.

6. Dentalvorrichtung nach Anspruch 1, wobei der Beschränkungsmechanismus einen Draht umfasst, der optional einen mittleren Teilabschnitt umfasst, der von einem polymeren Material umhüllt ist.

7. Dentalvorrichtung nach Anspruch 1, weiter umfassend ein bogenförmiges Stützelement (225) für erhöhte Steifigkeit oder Festigkeit der Vorrichtung, wobei sich das Element über die zweite Fläche erstreckt und nach oben und einer Kontur des Gaumens folgend angeordnet ist, ohne die Zähne oder den Gaumen der Person zu berühren, wenn die Vorrichtung getragen wird.

8. Dentalvorrichtung nach Anspruch 1, wobei die Komponente einen Draht mit einer mittleren Schnalle zur Flexibilität und optionalen seitlichen Ausdehnung der Komponente umfasst, wobei der Fixiermechanismus optional in eine der zweiten Flächen des Befestigungsmechanismus eingebettet ist, um den Fixiermechanismus am Befestigungsmechanismus anzubringen.

9. Dentalvorrichtung nach Anspruch 1, wobei der Fixiermechanismus eine Längseinstellung der Komponente nach vorne oder hinten ermöglicht.

10. Dentalvorrichtung nach Anspruch 6, wobei der Beschränkungsmechanismus über den Fixiermechanismus verschiebbar auf dem Einstellmechanismus montiert ist, um eine Einstellung der Position der Komponente entlang einer Längsachse der Vorrichtung zu ermöglichen.

11. Dentalvorrichtung nach Anspruch 1, wobei die Komponente des Beschränkungsmechanismus in Bezug auf die Okklusionsebene der Person beim Tragen der Vorrichtung oder auf eine durch die Enden des Beschränkungsmechanismus definierte Ebene abgewinkelt ist, wobei der Winkel im Bereich von etwa 0° bis etwa 30° oder +15° bis etwa -15° liegt, wenn die Vorrichtung von der Person getragen wird.

12. Dentalvorrichtung nach Anspruch 1, wobei die Komponente weiter einen Draht mit einer mittleren Schnalle zur Flexibilität und optionalen seitlichen Ausdehnung der Komponente umfasst.

13. Dentalvorrichtung nach Anspruch 1, wobei der Fixiermechanismus mindestens einen Fuß (422) umfasst, der an der entsprechenden zweiten Fläche des Befestigungsmechanismus angefügt oder eingebettet ist, um den Fixiermechanismus am Befestigungsmechanismus anzubringen, oder wobei der Fixiermechanismus eine Längseinstellung der Position der Komponente nach vorne und hinten ermöglicht.

## Revendications

1. Appareil dentaire destiné à limiter les mouvements d'une zone centrale ou postérieure de la langue d'une personne tout en permettant à une zone antérieure et à des bords latéraux de la langue d'effectuer les mouvements nécessaires à la parole et à la déglutition, l'appareil dentaire comprenant :
un mécanisme d'attache (200) destiné à attacher l'appareil dentaire sur les dents dans la bouche de la personne, dans lequel le mécanisme d'attache comprend une bande sensiblement, mais non totalement, circonférentielle (205) présentant :
une première surface (210) située sur un côté vestibulaire d'une arcade dentaire de la personne ;
une paire de secondes surfaces gauche et droite (215), chaque seconde surface étant opposée à la première surface et conçue pour être placée le long d'au moins une dent postérieure correspondante, gauche ou droite, du côté lingual de ladite au moins une dent, lesdites secondes surfaces étant conçues pour s'étendre le long du côté lingual d'un nombre limité de dents postérieures de la personne,
le mécanisme d'attache est conçu pour se mettre en prise sur l'arcade dentaire de la personne afin de fixer de manière amovible l'appareil sur l'arcade dentaire ;
un connecteur (220) reliant la première surface et la seconde surface ; et
un mécanisme de contrainte (300 ; 500) fixé au mécanisme d'attache et présentant un composant (305 ; 505) s'étendant entre les deux secondes surfaces, le composant présentant une orientation vers l'avant ou vers l'arrière et étant configuré pour se positionner approximativement au niveau du plan d'occlusion de la personne ou selon un angle par rapport à celui-ci, afin de limiter les mouvements de la zone centrale de la langue en orientation vers l'avant ou de la zone postérieure en orientation vers l'arrière, et ainsi restreindre l'amplitude des mouvements de la langue en la tirant vers l'avant et/ou vers le bas, tout en permettant au moins aux bords latéraux de la langue et, par conséquent, soit à sa zone postérieure et à son extrémité, soit à sa zone antérieure d'effectuer les mouvements nécessaires à la parole et à la déglutition ;
dans lequel, lorsque le composant présente une orientation vers l'avant, le mécanisme de contrainte est positionné au centre par rapport aux secondes surfaces et à une partie avant de la bande de manière à se loger dans une partie centrale de la langue ;
**caractérisé en ce que** le mécanisme de contrainte est fixé de manière amovible au mécanisme d'attache, et **en ce que** le mécanisme de contrainte ou le mécanisme d'attache comprend un mécanisme de réglage permettant d'ajuster : (i) l'angle du composant par rapport au plan occlusal de la personne lorsque l'appareil est porté et de commander ainsi l'étendue de la limitation du mouvement de la langue ; ou (ii) la position du composant le long d'un axe longitudinal de l'appareil et de commander ainsi la zone de la langue dans laquelle la contrainte doit être appliquée ; ou (iii) à la fois ledit angle et ladite position ;
dans lequel le mécanisme de contrainte est fixé au mécanisme d'attache par un mécanisme de fixation (400), dans lequel le mécanisme de fixation comprend un écrou et une vis ou une combinaison d'écrou et de boulon, dans lequel chaque extrémité du mécanisme de contrainte est montée et fixée de manière coulissante au mécanisme de fixation de manière réglable.

2. Appareil dentaire selon la revendication 1, dans lequel le composant s'étend entre les deuxièmes surfaces gauche et droite au niveau d'une partie arrière de la bande proche de la paire de secondes surfaces, éventuellement dans lequel le mécanisme de contrainte présente une extrémité reliée à la seconde surface gauche et une autre extrémité reliée à la seconde surface droite.

3. Appareil dentaire selon la revendication 1, dans lequel la bande sensiblement circonférentielle comprend un matériau résineux qui est en contact avec l'arcade dentaire lorsque l'appareil est porté, éventuellement, dans lequel la première surface du mécanisme d'attache épouse et est en alignement avec la ligne gingivale de la surface vestibulaire de l'arcade dentaire supérieure de la personne, éventuellement également, dans lequel la première surface du mécanisme d'attache couvre au moins la majeure partie de la surface vestibulaire de l'arcade dentaire à partir de la ligne gingivale mais ne couvre essentiellement pas la surface occlusale des dents de ladite arcade.

4. Appareil dentaire selon la revendication 1, dans lequel la bande sensiblement circonférentielle comprend en outre une ailette (235) attachée à la face linguale de la seconde surface, l'ailette portant un dispositif de détection, éventuellement intégré dans l'ailette, dans lequel le dispositif de détection est éventuellement un capteur de température, de pH ou de son, et comprenant en outre éventuellement au moins un des éléments suivants :
l'aile comprend un matériau résineux ; et
le dispositif de détection est intégré dans le matériau résineux et disposé de manière à ne pas toucher le palais ou les dents de la personne lorsque l'appareil est porté par la personne.

5. Appareil dentaire selon la revendication 1, dans lequel le composant est généralement en forme de C avec une section dorsale du composant disposée transversalement à un axe longitudinal de l'appareil et responsable de la limitation du mouvement de la langue, et dans lequel le mécanisme de contrainte présente une première et une seconde extrémité pour fixer le mécanisme de contrainte au mécanisme d'attache de la seconde surface correspondante via le mécanisme de fixation.

6. Appareil dentaire selon la revendication 1, dans lequel le mécanisme de contrainte comprend un fil comprenant éventuellement une section centrale enveloppée dans un matériau polymère.

7. Appareil dentaire selon la revendication 1, comprenant en outre un élément de support arqué (225) pour une rigidité ou une résistance accrue de l'appareil, dans lequel l'élément couvre les secondes surfaces et est disposé vers le haut et suit un contour du palais sans toucher les dents ou le palais de la personne lorsque l'appareil est porté.

8. Appareil dentaire selon la revendication 1, dans lequel le composant comprend un fil avec une boucle centrale pour la flexibilité et une expansion latérale optionnelle du composant, éventuellement, dans lequel le mécanisme de fixation est intégré dans l'une des secondes surfaces du mécanisme d'attache pour fixer le mécanisme de fixation au mécanisme d'attache.

9. Appareil dentaire selon la revendication 1, dans lequel le mécanisme de fixation permet d'ajuster longitudinalement la position du composant vers l'avant ou vers l'arrière.

10. Appareil dentaire selon la revendication 6, dans lequel le mécanisme de contrainte est monté de manière coulissante sur le mécanisme de réglage via le mécanisme de fixation pour permettre le réglage de la position du composant le long d'un axe longitudinal de l'appareil.

11. Appareil dentaire selon la revendication 1, dans lequel le composant du mécanisme de contrainte est incliné par référence au plan d'occlusion de la personne lorsque l'appareil est porté ou à un plan défini par les extrémités du mécanisme de contrainte, l'angle étant compris entre environ 0 et environ 30° ou +15° à environ -15° lorsque l'appareil est porté par la personne.

12. Appareil dentaire selon la revendication 1, dans lequel le composant comprend en outre un fil avec une boucle centrale pour la flexibilité et l'expansion latérale optionnelle du composant.

13. Appareil dentaire selon la revendication 1, dans lequel le mécanisme de fixation comprend au moins un pied (422) qui est fixé ou intégré dans la seconde surface correspondante du mécanisme d'attache pour fixer le mécanisme de fixation sur le mécanisme d'attache, ou, dans lequel le mécanisme de fixation permet d'ajuster longitudinalement la position du composant vers l'avant et vers l'arrière.
